# FASCICULE DE BREVET EUROPEEN

(11) **EP 3 226 850 B1**
(45) Date de publication et mention de la délivrance du brevet: **21.08.2019**
(21) Numéro de dépôt: 15821108.6
(22) Date de dépôt: 01.12.2015
(51) Int. Cl.: A61K 31/137, A61K 31/138, A61K 31/14, A61K 31/18, A61P 43/00, A61P 25/00

(54) **COMPOSES POUR LE TRAITEMENT DES MALADIES MITOCHONDRIALES**
VERBINDUNGEN ZUR BEHANDLUNG VON MITOCHONDRIOPATHIEN
COMPOUNDS FOR THE TREATMENT OF MITOCHONDRIAL DISEASES

(30) Priorité: 02.12.2014 FR 1461817
(43) Date de publication de la demande: 11.10.2017
(73) Titulaire: Université Paris-Sud, 91405 Orsay Cedex (FR); Centre National de la Recherche Scientifique, 75794 Paris Cedex 16 (FR); Universite D'Angers, 49035 Angers Cedex 01 (FR); Centre Hospitalier Universitaire d'Angers, 49333 Angers (FR)
(72) Inventeur: DELAHODDE, Agnès, 91370 Verrieres Le Buisson (FR); PITAYU NUGROHO, Laras, 91120 Palaiseau (FR); BARUFFINI, Enrico, 43123 Parme (IT); LODI, Tiziana, 43124 Parme (IT); RÖTIG, Agnès, 75007 Paris (FR); PROCACCIO, Vincent, 49240 Avrille (FR)
(74) Mandataire: Grosset-Fournier, Chantal Catherine
(86) Numéro de dépôt international: PCT/FR2015/053286
(87) Numéro de publication internationale: WO 2016/087771

(56) Documents cités:
- EP-A1- 0 164 865
- EP-A1- 2 243 476
- WO-A1-99/07832
- WO-A1-2012/112933
- WO-A1-2013/148740
- US-A- 4 289 787
- CARAMIA F ET AL: "Mitochondrial lesions of developing sympathetic neurons induced by bretylium tosylate", BRAIN RESEARCH, ELSEVIER, AMSTERDAM, NL, vol. 40, no. 2, 26 mai 1972 (1972-05-26), pages 237-246, XP024264641, ISSN: 0006-8993, DOI: 10.1016/0006-8993(72)90131-X [extrait le 1972-05-26]
- Laras Pitayu: "Les maladies mitochondriales liées à l'instabilité d'ADN mitochondrial : de la thérapie au mécanisme", , 28 septembre 2015 (2015-09-28), XP002753828, Extrait de l'Internet: URL:http://www.theses.fr/2015PA112233 [extrait le 2016-02-03]

## Description

L'invention a pour objet des composés pour leur utilisation dans le traitement et la prévention des maladies mitochondriales.

Les maladies mitochondriales regroupent un ensemble très large et hétérogène de maladies orphelines, qui comprend notamment les troubles liés à l'instabilité de l'ADN mitochondrial (ADNmt). Parmi eux, l'ophtalmoplégie externe progressive (Progressive External Ophthalmoplegia, PEO), l'insuffisance hépatique néonatale et la maladie d'Alpers (Alpers-Huttenlocher Syndrome, AHS) sont causées par des mutations dans le gène codant la polymérase gamma de l'ADN mitochondrial : le gène POLG (Spinazzola A, Zeviani M. Disorders of nuclear-mitochondrial intergenomic communication. Biosci Rep 2007;27(1-3):39-51).

Les mutations dans le gène POLG peuvent conduire à une accumulation de substitutions de bases, à des délétions de l'ADNmt ainsi qu'à des déplétions de l'ADNmt résultant en une production altérée de l'énergie via la phosphorylation oxydative.

Jusqu'à présent, plus de 200 mutations pathogéniques dans le gène POLG ont été reportées. Ces mutations sont associées à un très large spectre de maladies mitochondriales incluant entre autres la PEO, la myopathie, le parkinsonisme, la ménopause prématurée, les troubles psychologiques, l'ataxie, l'encéphalopathie et la maladie d'Alpers (Hudson G, Chinnery PF. Mitochondrial DNA polymerase-gamma and human disease. Hum Mol Genet. 2006;15:R244-52). Les mutations de POLG dominantes donnent lieu à une ophtalmoplégie externe progressive associée à des délétions multiples de l'ADNmt tandis que les mutations récessives provoquent une déplétion de l'ADNmt (2-10% de la quantité normale en ADNmt) et une insuffisance hépatique néonatale ou une maladie d'Alpers.

Il n'existe aujourd'hui aucun traitement curatif des maladies liées à l'instabilité de l'ADNmt. Les traitements sont actuellement limités à la gestion des symptômes et à des soins de soutien. Une grande variété de vitamines et cofacteurs a été utilisée chez des individus atteints de troubles mitochondriaux, bien qu'une revue systématique récente ait mis en évidence le manque de preuves justifiant leur utilisation (Chinnery P, Majamaa K, Turnbull D, Thorburn D. Treatment for mitochondrial disorders. Cochrane Database Syst Rev. 2006 Jan 25;(1):CD004426. Review. Update in: Cochrane Database Syst Rev. 2012;4:CD004426). La lévocarnitine, la créatine monohydrate, le coenzyme Q₁₀, les vitamines B et des antioxydants, tels que l'acide lipoïque alpha, la vitamine E et la vitamine C, ont été utilisés comme compléments mitochondriaux.

Ainsi, des thérapies à base de vitamines et cofacteurs peuvent être proposées dans le but de fortifier les fonctions mitochondriales. Cependant, il n'y a pas eu d'études formelles sur l'utilisation de ces vitamines et cofacteurs pour la maladie d'Alpers ou d'autres maladies liées au gène POLG (Parkih S, Saneto R, Falk MJ, Anslem I, Cohen BH, Haas R. A modem approach to the treament of mitochondrial diseases. Current Treatment Options in Neurology. 2009;11:414-430). Il a également été rapporté que l'emploi de la lévo-arginine aide à réduire la fréquence et la sévérité des accidents vasculaires cérébraux associés au syndrome MELAS et pourrait être considéré chez les patients atteints de maladies liées à POLG, en particulier si une carence dans le plasma ou une concentration d'arginine dans le liquide céphalorachidien est confirmé.

Le recours à des traitements de l'épilepsie réfractaire tels que la corticotropine ou la prednisone, le régime cétogène et les immunoglobulines G intraveineuses n'a pas été démontré être efficace pour le traitement de l'AHS.

Une transplantation du foie chez les adultes souffrant de l'AHS et possédant une qualité de vie acceptable peut être bénéfique. Toutefois, la transplantation du foie n'est pas conseillée pour les enfants, celle-ci n'ayant pas d'effet sur la progression rapide de l'atteinte neurologique (Kelly DA. Liver transplantation: to do or not to do? Pediatr Transplant. 2000;4:170-2).

Le clofilium tosylate est un agent antiarythmique de classe III dont le mécanisme d'action repose sur le blocage des canaux potassium, augmentant ainsi la durée des potentiels d'action cardiaque (US 4 289 787).

L'Ibutilide est un agent antiarythmique de classe III commercialisé sous le nom de Corvert (sous forme de fumarate d'ibutilide) par Pfizer. Ce médicament est utilisé pour la réduction rapide de la fibrillation ou du flutter auriculaire en rythme sinusal. Il agit en retardant la repolarisation par activation d'un courant lent entrant essentiellement sodique (EP 0 164 865).

Le Dofétilide est un antiarythmique de classe III commercialisé par Pfizer sous le nom Tikosyn. Il est prescrit pour la prise en charge des fibrillations et flutters auriculaires. Il agit en bloquant les canaux potassium (EP 0 245 997).

L'un des buts de l'invention est de fournir des composés efficaces dans le traitement des maladies mitochondriales.

Un autre aspect de l'invention est de fournir des composés efficaces dans le traitement de pathologies résultant de mutations dans le gène POLG.

Un autre aspect de l'invention est de fournir des composés efficaces dans le traitement du syndrome MELAS.

L'un des avantages de l'invention est de fournir des composés efficaces chez l'adulte et chez l'enfant.

Un autre avantage de l'invention est de fournir des composés permettant un traitement curatif des maladies mitochondriales et ne se limitant pas à la gestion des symptômes des maladies mitochondriales.

La présente divulgation décrit un composé ayant la formule I 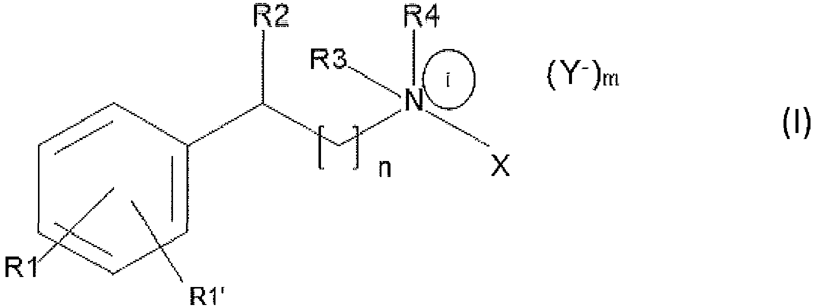 dans laquelle
R₁ et R_{1'} représentent indépendamment l'un de l'autre un atome d'hydrogène, un atome d'halogène, notamment Cl ou Br, un groupe alcoxy en C1-C4, un groupe alkyle en C1-C3 ou NHSO₂R₆, sous réserve qu'au moins l'un des deux éléments R₁ ou R_{1'} soit différent de H ; R₂ représente OH ou H ;
R₃ et R₄ représentent indépendamment l'un de l'autre un groupe alkyle de 1 à 10 atomes de carbone ou 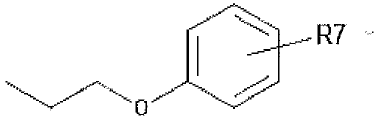 où R₇ représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
-X=-R₅ ou le doublet électronique de l'azote non engagé dans une liaison, sous réserve que i = + lorsque -X=-R₅ ;
R₅ représente un atome d'hydrogène ou un groupe alkyle en C1-C4;
m = 0 ou 1, sous réserve que m = 1 lorsque -X = -R₅ et m = 0 lorsque -X = le doublet électronique de l'azote non engagé dans une liaison ; n= 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

Ø représente l'ensemble vide.

La présente divulgation décrit en particulier un composé de formule I 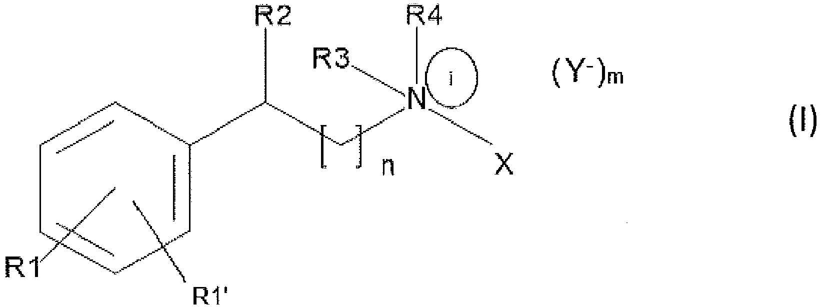 dans laquelle
R₁ et R_{1'} représentent indépendamment l'un de l'autre un atome d'hydrogène, un atome d'halogène, notamment Cl ou Br, un groupe alcoxy en C1-C4, un groupe alkyle en C1-C3 ou NHSO₂R₆, sous réserve qu'au moins l'un des deux éléments R₁ ou R_{1'} soit différent de H;
R₂ représente OH ou H ;
R₃ et R₄ représentent indépendamment l'un de l'autre un groupe alkyle de 1 à 10 atomes de carbone ou 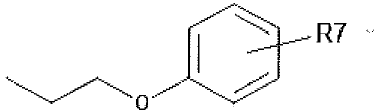 où R₇ représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
-X=-R₅ ou le doublet électronique de l'azote non engagé dans une liaison, sous réserve que i = + lorsque-X = -R₅ ;
R₅ représente un groupe alkyle en C1-C4;
m = 0 ou 1, sous réserve que m = 1 lorsque -X = - R₅ et m = 0 lorsque -X = 1e doublet électronique de l'azote non engagé dans une liaison ;
n= 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

Dans un mode de réalisation particulier de la divulgation, le composé de formule I tel que défini ci-dessus, pour son utilisation dans le traitement ou la prévention des maladies liées à l'instabilité de l'ADN mitochondrial, comporte un élément Y⁻ choisi parmi l'ion tosylate, l'ion carbonate, l'ion phosphate et l'ion chlorure.

Avantageusement, la présente divulgation concerne un composé de formule I tel que défini ci-dessus 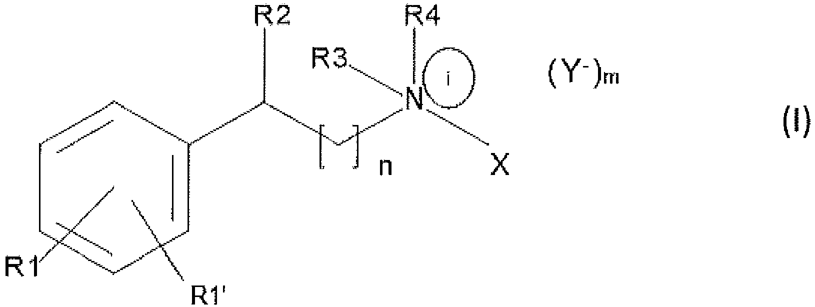 formule dans laquelle :
R₁ et R_{1'} représentent indépendamment l'un de l'autre un atome d'hydrogène, Cl, Br, ou NHSO₂R₆, sous réserve qu'au moins l'un des deux éléments R1 ou R1' soit différent de H;
R₂ représente OH ou H ;
R₃ et R₄ représentent indépendamment l'un de l'autre un groupe alkyle de 1 à 10 atomes de carbone ou 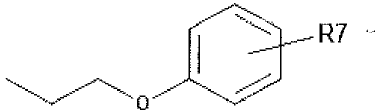 où R₇ représente un atome d'halogène ou NHSO₂R₈, sous réserve que R₃ et R₄ ne peuvent pas être simultanément 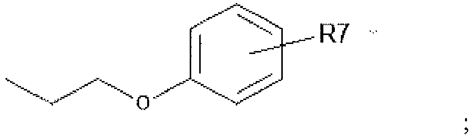
R₆ et R₈ représentent indépendamment l'un de l'autre un groupe alkyle en C1-C4 ;
-X =-R₅ ou le doublet électronique de l'azote non engagé dans une liaison, sous réserve que i = + lorsque -X = -R₅ ;
R₅ représente un groupe alkyle en C1-C4;
m = 0 ou 1, sous réserve que m = 1 lorsque -X = - R₅ et m = 0 lorsque -X = le doublet électronique de l'azote non engagé dans une liaison ;
n=0, 1, 2 ou 3 ;
Y⁻ est choisi parmi l'ion tosylate, l'ion carbonate, l'ion phosphate et l'ion chlorure ;
pour son utilisation dans le traitement ou la prévention des maladies liées à l'instabilité de l'ADN mitochondrial.

La présente invention concerne un composé ayant la formule la : 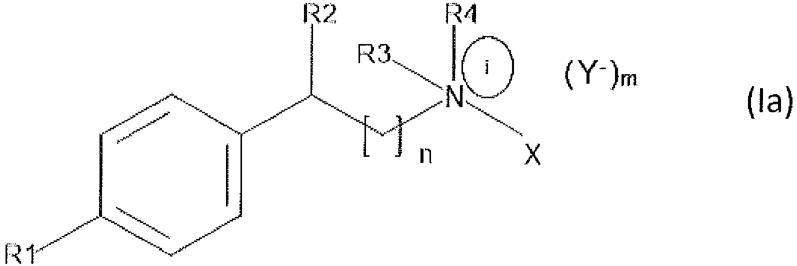 dans laquelle
R₁ représente un atome d'halogène, notamment Cl ou Br, un groupe alcoxy en C1-C4, un groupe alkyle en C1-C3 ou NHSO₂R₆;
R₂ représente OH ou H ;
R₃ et R₄ représentent indépendamment l'un de l'autre un groupe alkyle de 1 à 10 atomes de carbone ou 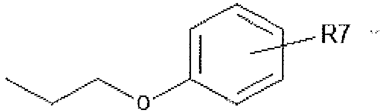 où R₇ représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
-X =-R₅ ou le doublet électronique de l'azote non engagé dans une liaison, sous réserve que i = + lorsque -X =-R₅ ;
R₅ représente un atome d'hydrogène ou un groupe alkyle en C1-C4;
m = 0 ou 1, sous réserve que m = 1 lorsque -X = -R₅ et m = 0 lorsque -X = le doublet électronique de l'azote non engagé dans une liaison ;
n= 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

Les inventeurs ont trouvé de façon inattendue que l'application des composés de l'invention à des organismes atteints d'une maladie mitochondriale permettait une amélioration de leur état. Cette invention s'appuie notamment sur des résultats obtenus en appliquant les composés définis ci-dessus à des organismes mimant une maladie mitochondriale ou à des cultures cellulaires de fibroblastes cutanés d'un patient atteint d'une maladie mitochondriale ou à des cultures cellulaires de cybrides neuronaux dérivés d'un patient atteint d'une maladie mitochondriale.

Le terme « groupe alcoxy en Cx-Cy » signifie un groupe alkyle, possédant de x à y atomes de carbone, lié à un atome d'oxygène.

Le terme « groupe alkyle en Cx-Cy » signifie une chaine hydrocarbonée saturée, linéaire ou branchée, comprenant de x à y atomes de carbone.

Par « X = - R₅ », on entend que l'amine à laquelle est liée le groupe R₅ est une amine quaternaire et que dans ce cas i = + et m = 1, le composé étant alors sous forme de sel.

Par « X = le doublet électronique de l'azote non engagé dans une liaison », on entend que l'amine correspondante est une amine tertiaire et que dans ce cas i = Ø, m = 0 et (Y⁻)ₘ = Ø.

Par « amine quaternaire », on entend que l'atome d'azote est chargé positivement, c'est-à-dire qu'il est sous forme de cation, et qu'il est substitué par 4 substituants. Au sens de la présente invention, l'un des substituants peut être l'atome d'hydrogène.

Par « amine tertiaire », on entend que l'atome d'azote est substitué par 3 substituants.

Par « anion acceptable sur le plan thérapeutique », on entend tout anion non toxique et n'induisant pas une perte de l'effet thérapeutique ou prophylactique du cation auquel il est associé.

Par « instabilité de l'ADN mitochondrial », on entend la présence de toutes mutations, délétions et/ou déplétions de l'ADN mitochondrial par rapport à l'ADN mitochondrial d'un individu sain.

Au sens de la présente invention, on entend par « maladies liées à l'instabilité de l'ADN mitochondrial » les maladies liées à l'aspect quantitatif de l'instabilité de l'ADN mitochondrial et les maladies liées à l'aspect qualitatif de l'instabilité de l'ADN mitochondrial.

Par « maladies liées à l'aspect quantitatif de l'instabilité de l'ADN mitochondrial », on entend les maladies liées à une déplétion de l'ADN mitochondrial. Cette déplétion peut notamment être due à une ou des mutations présentes dans le gène POLG, un gène nucléaire codant pour la polymérase POLG spécifique à l'ADN mitochondrial.

Par « déplétion de l'ADN mitochondrial », on entend une réduction du nombre de copies de molécules d'ADN mitochondrial dans les mitochondries d'un organisme par rapport à un organisme sain. Les déplétions sont tissu spécifiques.

Par « maladies liées à l'aspect qualitatif de l'instabilité de l'ADN mitochondrial », on entend les maladies dont la cause réside en la présence de délétions et/ou de mutations dans l'ADN mitochondrial.

L'expression « délétion de l'ADN mitochondrial» signifie une perte de matériel génétique dans l'ADN mitochondrial par rapport à un ADN mitochondrial sauvage. Une délétion peut concerner un ou plusieurs nucléotides, notamment plusieurs centaines de nucléotides, de l'ADN mitochondrial.

L'expression « mutation de l'ADN mitochondrial » signifie une modification de l'ADN mitochondrial par rapport à un ADN mitochondrial sauvage. Les mutations regroupent les mutations par substitution, les mutations par insertion et les mutations par délétion de petite taille (de 1 à 10 nucléotides). Le terme « mutation par substitution » signifie le remplacement d'un ou plusieurs nucléotides par un nucléotide différent dans la molécule d'ADN mitochondrial. Le terme « mutation par insertion » signifie l'addition d'un ou de plusieurs nucléotides dans la molécule d'ADN mitochondrial.

Les maladies liées à l'instabilité de l'ADN mitochondrial comprennent notamment, sans y être limitées, le syndrome d'Alpers-Huttenlocher les (AHS), le spectre des myo-cérébro-hépatopathies de l'enfant (MCHS), les épilepsies myocloniques avec myopathie et ataxie sensitive MEMSA (Myoclonic epilepsy myopathy sensory ataxia), l'ataxie spinocérébelleuse avec épilepsie (SCAE), les ataxies avec neuropathies incluant les syndromes MIRAS et SANDO, l'ophtalmoplégie externe progressive autosomique récessive (arPEO), l'ophtalmoplégie externe progressive autosomique dominante (adPEO), l'encéphalomyopathie mitochondriale neurogastrointestinale (MNGIE), la myopathie infantile et amyotrophie spinale liées aux mutations de *TK2*, les insuffisances hépatiques avec déplétion de l'ADNmt, les pathologies associées aux mutations des gènes *SUCLA2* et *SUCLAG1*, *RRM2B*, *AIF1*, *MPV17*, la neuropathie optique héréditaire de Leber, le syndrome MELAS, le syndrome MERRF, ainsi que certaines formes du syndrome de Leigh, d'ophtalmoplégie externe progressive chronique, de myopathie, de cardiomyopathie, de diabète-surdité, d'encéphalomyopathie, et de surdité.

Dans un mode de réalisation particulier de l'invention, le composé de formule la tel que défini ci-dessus, pour son utilisation dans le traitement ou la prévention des maladies liées à l'instabilité de l'ADN mitochondrial, comporte un élément Y⁻ choisi parmi l'ion tosylate, l'ion carbonate, l'ion phosphate et l'ion chlorure.

Dans un mode de réalisation particulier, l'invention concerne un composé tel que défini ci-dessus, de formule la : 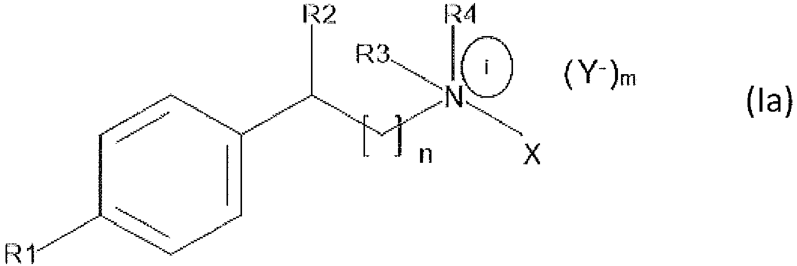 dans laquelle
R₁ représente Cl, Br, ou NHSO₂R₆;
R₂ représente OH ou H ;
R₃ et R₄ représentent indépendamment l'un de l'autre un groupe alkyle de 1 à 10 atomes de carbone ou 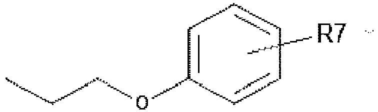 où R₇ représente un atome d'halogène ou NHSO₂R₈, sous réserve que R₃ et R₄ ne peuvent pas être simultanément 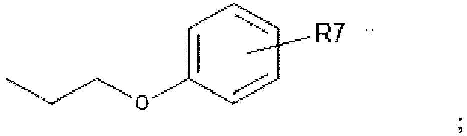
R₆ et R₈ représentent indépendamment l'un de l'autre un groupe alkyle en C1-C4 ;
-X=-R₅ ou le doublet électronique de l'azote non engagé dans une liaison, sous réserve que i = + lorsque -X=-R₅ ;
R₅ représente un groupe alkyle en C1-C4;
m = 0 ou 1, sous réserve que m = 1 lorsque -X=-R₅ et m = 0 lorsque -X = le doublet électronique de l'azote non engagé dans une liaison ;
n = 0, 1, 2 ou 3 ;
Y⁻ est choisi parmi l'ion tosylate, l'ion carbonate, l'ion phosphate et l'ion chlorure ;
pour son utilisation dans le traitement ou la prévention des maladies liées à l'instabilité de l'ADN mitochondrial.

Dans un mode de réalisation particulier, l'invention concerne un composé tel que défini ci-dessus, de formule Iaa : 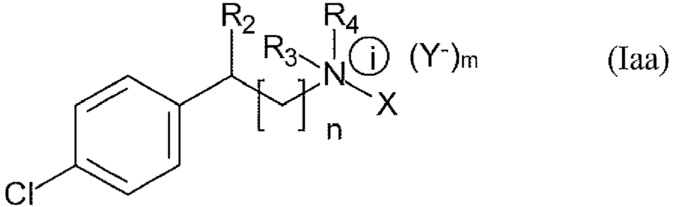 dans laquelle
R₂ représente OH ou H ;
R₃ et R₄ représentent indépendamment l'un de l'autre un groupe alkyle de 1 à 10 atomes de carbone ou 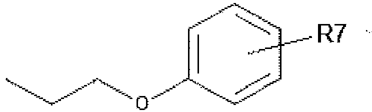 où R₇ représente un atome d'hydrogène, un atome d'halogène, un groupe alcoxy en C1-C4, un groupe alkyle en C1-C3 ou NHSO₂R₈ ;
R₈ représente un groupe alkyle en C1-C4 ;
-X=-R₅ ou le doublet électronique de l'azote non engagé dans une liaison, sous réserve que i = + lorsque -X=-R₅ ;
R₅ représente un atome d'hydrogène ou un groupe alkyle en C1-C4;
m = 0 ou 1, sous réserve que m = 1 lorsque -X=-R₅ et m = 0 lorsque -X= le doublet électronique de l'azote non engagé dans une liaison ;
n= 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

Dans un autre mode de réalisation particulier, l'invention concerne un composé tel que défini ci-dessus, de formule Iab : 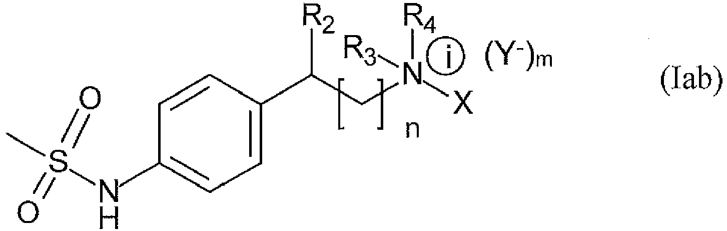 dans laquelle
R₂ représente OH ou H ;
R₃ et R₄ représentent indépendamment l'un de l'autre un groupe alkyle de 1 à 10 atomes de carbone ou 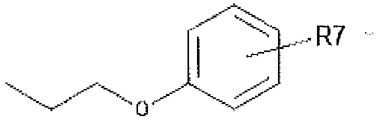 où R₇ représente un atome d'hydrogène, un atome d'halogène, un groupe alcoxy en C1-C4, un groupe alkyle en C1-C3 ou NHSO₂R₈ ;
Rg représente un groupe alkyle en C1-C4 ;
-X=-R₅ ou le doublet électronique de l'azote non engagé dans une liaison, sous réserve que i = + lorsque -X =-R₅ ;
R₅ représente un atome d'hydrogène ou un groupe alkyle en C1-C4;
m = 0 ou 1, sous réserve que m = 1 lorsque -X =-R₅ et m = 0 lorsque -X = le doublet électronique de l'azote non engagé dans une liaison ;
n= 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

La présente divulgation décrit également un composé de formule Ib 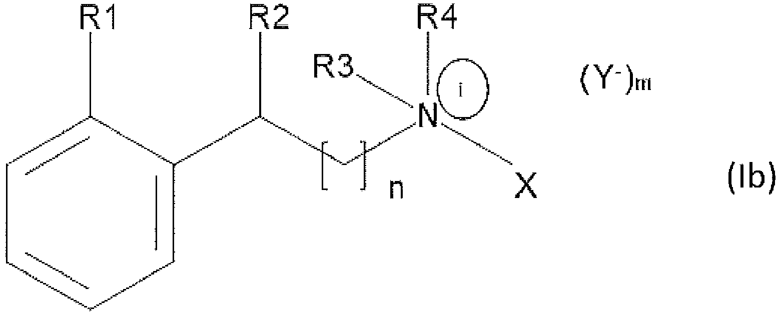 dans laquelle
R₁ représente un atome d'halogène, notamment Cl ou Br, un groupe alcoxy en C1-C4, un groupe alkyle en C1-C3 ou NHSO₂R₆;
R₂ représente OH ou H ;
R₃ et R₄ représentent indépendamment l'un de l'autre un groupe alkyle de 1 à 10 atomes de carbone ou 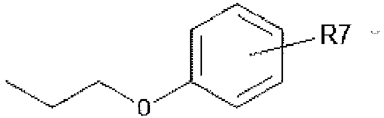 où R₇ représente un atome d'hydrogène, un atome d'halogène, un groupe alcoxy en C1-C4, un groupe alkyle en C1-C3 ou NHSO₂R₈;
R₆ et R₈ représentent indépendamment l'un de l'autre un groupe alkyle en C1-C4 ;
-X=-R₅ ou le doublet électronique de l'azote non engagé dans une liaison, sous réserve que i = + lorsque -X=-R₅ ;
R₅ représente un atome d'hydrogène ou un groupe alkyle en C1-C4;
m = 0 ou 1, sous réserve que m = 1 lorsque -X = - R₅ et m = 0 lorsque -X = le doublet électronique de l'azote non engagé dans une liaison ;
n = 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

La présente divulgation décrit également un composé de formule Ic 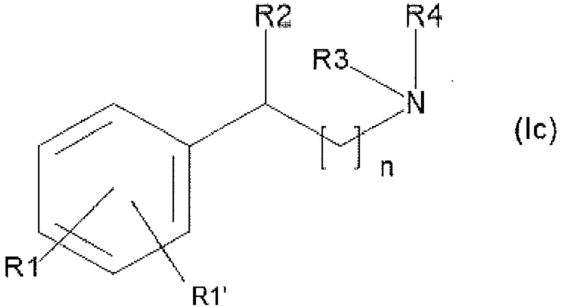 dans laquelle
R₁ et R_{1'} représentent indépendamment l'un de l'autre un atome d'hydrogène, un atome d'halogène, notamment Cl ou Br, un groupe alcoxy en C1-C4, un groupe alkyle en C1-C3 ou NHSO₂R₆, sous réserve qu'au moins l'un des deux éléments R₁ ou R_{1'} soit différent de H;
R₂ représente OH ou H ;
R₃ et R₄ représentent indépendamment l'un de l'autre un groupe alkyle de 1 à 10 atomes de carbone ou 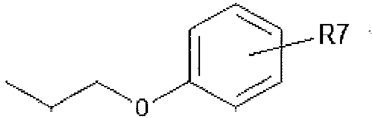 où R7 représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
n = 0, 1, 2 ou 3 ;
pour son utilisation dans le traitement ou la prévention des maladies liées à l'instabilité de l'ADN mitochondrial.

Le groupement amine supportant les groupes R₃ et R₄ du composé de formule Ic est une amine tertiaire.

Dans un autre mode de réalisation particulier, la présente divulgation décrit un composé de formule Id 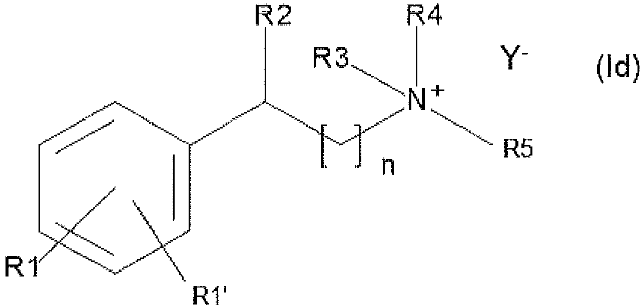 dans laquelle
R₁ et R_{1'} représentent indépendamment l'un de l'autre un atome d'hydrogène, un atome d'halogène, notamment Cl ou Br, un groupe alcoxy en C1-C4, un groupe alkyle en C1-C3 ou NHSO₂R₆, sous réserve qu'au moins l'un des deux éléments R₁ ou R_{1'} soit différent de H;
R₂ représente OH ou H ;
R₃ et R₄ représentent indépendamment l'un de l'autre un groupe alkyle de 1 à 10 atomes de carbone ou 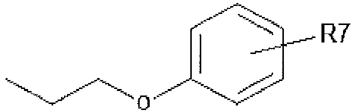 où R₇ représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
R₅ représente un atome d'hydrogène ou un groupe alkyle en C₁-C₄;
n = 0, 1, 2 ou 3 ;
Y⁻ est un anion acceptable sur le plan thérapeutique;
pour son utilisation dans le traitement ou la prévention des maladies liées à l'instabilité de l'ADN mitochondrial.

Le groupement amine supportant les groupes R₃, R₄ et R₅ du composé de formule Id est une amine quaternaire.

Dans un mode de réalisation avantageux, le composé de l'invention tel que défini ci-dessus a pour formule (a) :

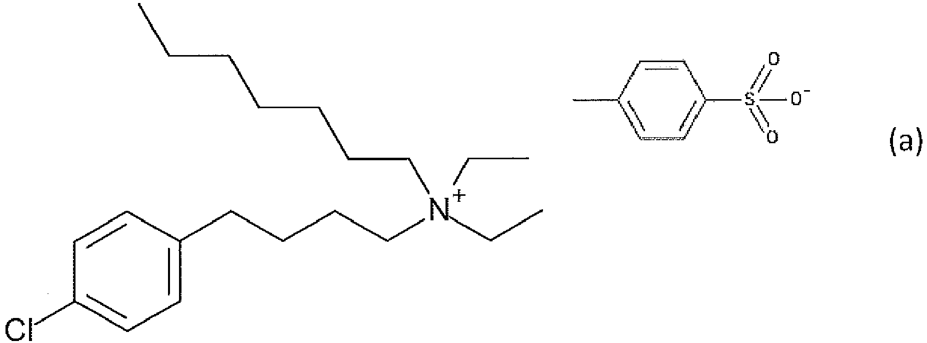

Le composé de formule (a) est le clofilium tosylate, le composé de formule : 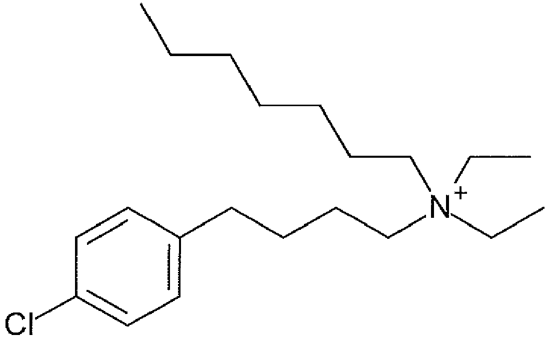 étant le clofilium,
et le composé de formule : 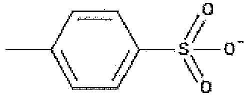 étant l'ion tosylate.

Dans un autre mode de réalisation avantageux, le composé de l'invention tel que défini ci-dessus a pour formule (b) :

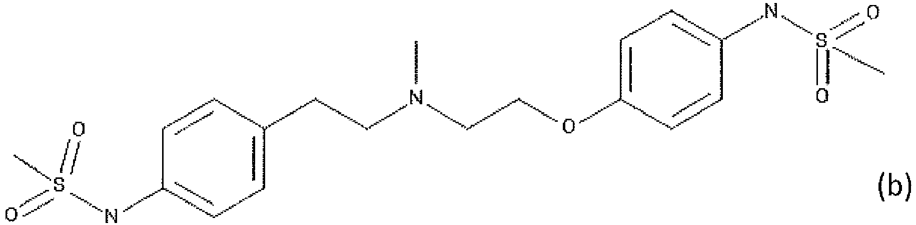

Le composé de formule (b) est le Dofétilide.

Dans un autre mode de réalisation avantageux, le composé de l'invention tel que défini ci-dessus a pour formule (c):

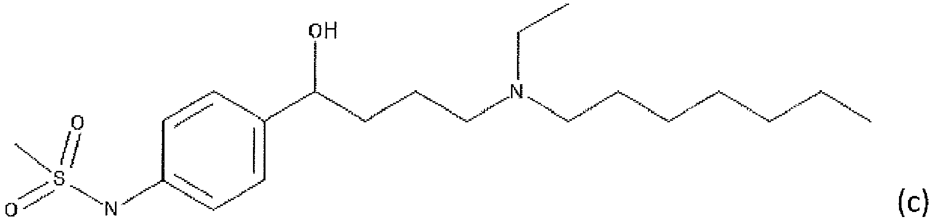

Le composé de formule (c) est l'Ibutilide.

Dans un mode de réalisation particulier, l'invention concerne un composé de formule (a), (b) ou (c), pour une administration à une dose de 1 µg/kg à 50 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration à une dose de 1 à 50 µg/kg, de 50 à 500 µg/kg, de 500 µg à 5 mg/kg, ou de 5 à 50 mg/kg, encore plus particulièrement à une dose de 1 à 12 µg/kg, de 12 à 20 µg/kg, de 20 à 50 µg/kg, de 20 à 300 µg/kg, de 50 à 300 µg/kg, de 300 à 500 µg/kg , de 500 µg/kg à 1 mg/kg, de 1 à 5 mg/kg, de 5 à 30 mg/kg, ou de 30 à 50 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 30 µg à 6000 mg, pour son utilisation dans le traitement ou la prévention des maladies liées à l'instabilité de l'ADN mitochondrial.

Au sens de la présente invention, on entend par « dose unitaire » la dose correspondant à la dose totale du composé prise en une seule fois, quel que soit le mode d'administration du composé.

Plus particulièrement, l'invention concerne un composé de formule (a), (b) ou (c), se présentant sous forme de dose unitaire de 30 à 500 µg, de 500 µg à 2,5 mg, de 2,5 à 60 mg, de 60 à 150 mg, ou de 150 à 6000 mg, encore plus particulièrement se présentant sous forme de dose unitaire de 30 à 150 µg, de 150 à 300 µg, de 300 à 500 µg, de 500 µg à 1 mg, de 1 à 2,5 mg, de 2,5 à 30 mg, de 30 mg à 60 mg, de 150 à 2500 mg, de 2500 à 4500 mg ou de 4500 à 6000 mg, pour son utilisation dans le traitement ou la prévention des maladies liées à l'instabilité de l'ADN mitochondrial.

Dans un mode de réalisation particulier, l'invention concerne un composé de formule (a), (b) ou (c), pour une administration par voie orale à une dose de 1 µg/kg à 50 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration par voie orale à une dose de 1 à 50 µg/kg, de 50 à 500 µg/kg, de 500 µg/kg à 5 mg/kg, ou de 5 à 50 mg/kg, encore plus particulièrement à une dose de 1 à 20 µg/kg, de 20 à 50 µg/kg, de 50 à 300 µg/kg, de 300 à 500 µg/kg, de 500 µg/kg à 1 mg/kg, de 1 à 5 mg/kg, de 5 à 30 mg/kg, ou de 30 à 50 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 30 µg à 6000 mg, pour une administration par voie orale, pour son utilisation dans le traitement ou la prévention des maladies liées à l'instabilité de l'ADN mitochondrial.

Plus particulièrement, l'invention concerne un composé de formule (a), (b) ou (c), se présentant sous forme de dose unitaire de 30 à 500 µg, de 500 µg à 60 mg, ou de 60 mg à 6000 mg, encore plus particulièrement se présentant sous forme de dose unitaire de 30 à 125 µg, de 125 à 300 µg, de 300 à 500 µg, de 500 µg à 1,5 mg, de 1,5 à 25 mg, de 25 à 60 mg, de 60 à 500 mg, de 500 à 2000 mg, ou de 2000 à 6000 mg, pour une administration par voie orale, pour son utilisation dans le traitement ou la prévention des maladies liées à l'instabilité de l'ADN mitochondrial.

Dans un autre mode de réalisation particulier, l'invention concerne un composé de formule (a), (b) ou (c), pour une administration par voie intraveineuse à une dose de 1 µg/kg à 50 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration par voie intraveineuse à une dose de 1 à 20 µg/kg, de 20 à 500 µg/kg, de 500 µg/kg à 5 mg/kg, ou de 5 à 50 mg/kg, encore plus particulièrement à une dose de 1 à 12 µg/kg, de 12 à 20 µg/kg, de 20 à 50 µg/kg, de 20 à 150 µg/kg, de 50 à 300 µg/kg, de 300 à 500 µg/kg, de 500 µg/kg à 1 mg/kg, de 1 à 5 mg/kg, de 5 à 30 mg/kg, ou de 30 à 50 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 30 µg à 6000 mg, pour une administration par voie intraveineuse, pour son utilisation dans le traitement ou la prévention des maladies liées à l'instabilité de l'ADN mitochondrial.

Plus particulièrement, l'invention concerne un composé de formule (a), (b) ou (c), se présentant sous forme de dose unitaire de 30 à 500 µg, de 500 µg à 30 mg, de 30 à 150 mg, ou de 150 à 6000 mg, encore plus particulièrement se présentant sous forme de dose unitaire de 30 à 150 µg, de 150 à 500 µg, de 500 µg à 1,5 mg, de 1,5 à 30 mg, de 30 à 60 mg, de 60 à 150 mg, de 150 à 1000 mg, de 1000 à 2500 mg, ou de 2500 à 6000 mg, pour une administration par voie intraveineuse, pour son utilisation dans le traitement ou la prévention des maladies liées à l'instabilité de l'ADN mitochondrial.

Dans un autre mode de réalisation particulier, l'invention concerne un composé de formule (a), (b) ou (c), pour une administration à une dose de 0,1 µg/kg à 5 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration à une dose de 0,1 à 5 µg/kg, de 5 à 50 µg/kg, de 50 µg/kg à 0,5 mg/kg, ou de 0,5 à 5 mg/kg, encore plus particulièrement à une dose de 0,1 à 1,2 µg/kg, de 1,2 à 2 µg/kg, de 2 à 5 µg/kg, de 2 à 30 µg/kg, de 5 à 30 µg/kg, de 30 à 50 µg/kg, de 50 µg/kg à 100 µg/kg, de 100 à 500 µg/kg, de 500 µg/kg à 3 mg/kg , ou de 3 à 5 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 3 µg à 600 mg, pour son utilisation dans le traitement ou la prévention des maladies liées à l'instabilité de l'ADN mitochondrial.

Plus particulièrement, l'invention concerne un composé de formule (a), (b) ou (c), se présentant sous forme de dose unitaire de 3 à 50 µg, de 50 µg à 250 µg, de 250 µg à 6 mg, de 6 à 15 mg, ou de 15 à 600 mg, encore plus particulièrement se présentant sous forme de dose unitaire de 3 à 15 µg, de 15 à 30 µg, de 30 à 50 µg, de 50 µg à 0,1 mg, de 0,1 à 0,25 mg, de 0,25 à 3 mg, de 3 mg à 6 mg, de 15 à 250 mg, de 250 à 450 mg ou de 450 à 600 mg, pour son utilisation dans le traitement ou la prévention des maladies liées à l'instabilité de l'ADN mitochondrial.

Dans un mode de réalisation particulier, l'invention concerne un composé de formule (a), (b) ou (c), pour une administration par voie orale à une dose de 0,1 µg/kg à 5 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration par voie orale à une dose de 0,1 à 5 µg/kg, de 5 à 50 µg/kg, de 50 µg/kg à 0,5 mg/kg, ou de 0,5 à 5 mg/kg, encore plus particulièrement à une dose de 0,1 à 2 µg/kg, de 2 à 5 µg/kg, de 5 à 30 µg/kg, de 30 à 50 µg/kg, de 50 µg/kg à 100 µg/kg, de 100 µg/kg à 500 µg/kg, de 500 µg/kg à 3 mg/kg, ou de 3 à 5 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 3 µg à 600 mg, pour une administration par voie orale, pour son utilisation dans le traitement ou la prévention des maladies liées à l'instabilité de l'ADN mitochondrial.

Plus particulièrement, l'invention concerne un composé de formule (a), (b) ou (c), se présentant sous forme de dose unitaire de 3 à 50 µg, de 50 µg à 6 mg, ou de 6 mg à 600 mg, encore plus particulièrement se présentant sous forme de dose unitaire de 3 à 12,5 µg, de 12,5 à 30 µg, de 30 à 50 µg, de 50 µg à 150 µg, de 150 µg à 2,5 mg, de 2,5 à 6 mg, de 6 à 50 mg, de 50 à 200 mg, ou de 200 à 600 mg, pour une administration par voie orale, pour son utilisation dans le traitement ou la prévention des maladies liées à l'instabilité de l'ADN mitochondrial.

Dans un autre mode de réalisation particulier, l'invention concerne un composé de formule (a), (b) ou (c), pour une administration par voie intraveineuse à une dose de 0,1 µg/kg à 5 mg/kg, pour son utilisation dans le traitement ou la prévention des maladies liées à l'instabilité de l'ADN mitochondrial.

Plus particulièrement, l'invention concerne un composé de formule (a), (b) ou (c), pour une administration par voie intraveineuse à une dose de 0,1 à 2 µg/kg, de 2 à 50 µg/kg, de 50 µg/kg à 0,5 mg/kg, ou de 0,5 à 5 mg/kg, encore plus particulièrement à une dose de 0,1 à 1,2 µg/kg, de 1,2 à 2 µg/kg, de 2 à 5 µg/kg, de 2 à 15 µg/kg, de 5 à 30 µg/kg, de 30 à 50 µg/kg, de 50 µg/kg à 100 µg/kg, de 100 à 500 µg/kg, de 500 µg/kg à 3 mg/kg, ou de 3 à 5 mg/kg, pour son utilisation dans le traitement ou la prévention des maladies liées à l'instabilité de l'ADN mitochondrial.

Dans un autre mode de réalisation particulier, l'invention concerne un composé de formule (a), (b) ou (c), se présentant sous forme de dose unitaire de 3 µg à 600 mg, pour une administration par voie intraveineuse, pour son utilisation dans le traitement ou la prévention des maladies liées à l'instabilité de l'ADN mitochondrial.

Plus particulièrement, l'invention concerne un composé de formule (a), (b) ou (c), se présentant sous forme de dose unitaire de 3 à 50 µg, de 50 µg à 3 mg, de 3 à 15 mg, ou de 15 à 600 mg, encore plus particulièrement se présentant sous forme de dose unitaire de 3 à 15 µg, de 15 à 50 µg, de 50 µg à 0,15 mg, de 0,15 à 3 mg, de 3 à 6 mg, de 6 à 15 mg, de 15 à 100 mg, de 100 à 250 mg, ou de 250 à 600 mg, pour une administration par voie intraveineuse, pour son utilisation dans le traitement ou la prévention des maladies liées à l'instabilité de l'ADN mitochondrial.

Dans un mode de réalisation encore plus particulier, l'invention concerne un composé de formule (a) : 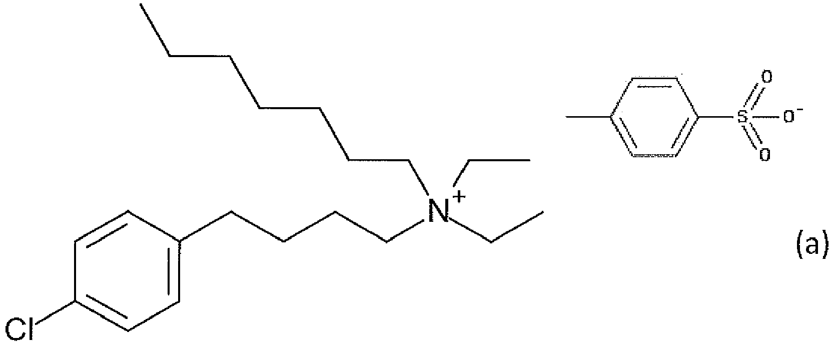 pour une administration par voie orale à une dose de 50 à 500 µg/kg, pour son utilisation dans le traitement ou la prévention des maladies liées à l'instabilité de l'ADN mitochondrial.

Plus particulièrement, l'invention concerne un composé de formule (a), pour une administration par voie orale à une dose de 50 à 300 µg/kg, ou de 300 à 500 µg/kg, encore plus particulièrement à une dose de 50 à 100 µg/kg, de 100 à 200 µg/kg, de 200 à 300 µg/kg, de 300 à 400 µg/kg ou de 400 à 500 µg/kg, pour son utilisation dans le traitement ou la prévention des maladies liées à l'instabilité de l'ADN mitochondrial.

Dans un autre mode de réalisation particulier, l'invention concerne un composé de formule (a) : 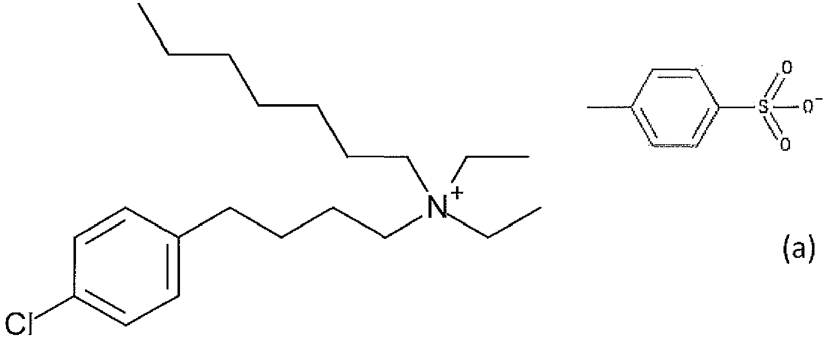 se présentant sous forme de dose unitaire de 1,5 mg à 60 mg, pour une administration par voie orale, pour son utilisation dans le traitement ou la prévention des maladies liées à l'instabilité de l'ADN mitochondrial.

Plus particulièrement, l'invention concerne un composé de formule (a), se présentant sous forme de dose unitaire de 1,5 à 25 mg ou de 25 à 60 mg, encore plus particulièrement de 1,5 à 8 mg, de 8 à 15 mg, de 15 à 25 mg, de 25 à 35 mg, de 35 à 60 mg, de 35 à 45 mg, ou de 45 à 60 mg, pour une administration par voie orale, pour son utilisation dans le traitement ou la prévention des maladies liées à l'instabilité de l'ADN mitochondrial.

Dans un autre mode de réalisation particulier, l'invention concerne un composé de formule (a) : 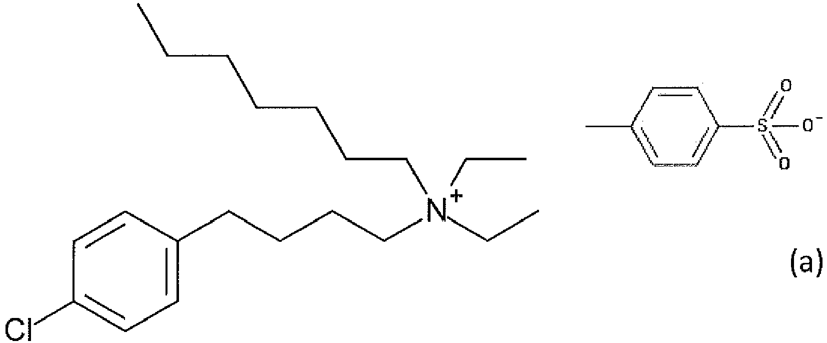 pour une administration par voie intraveineuse à une dose de 20 à 250 µg/kg, pour son utilisation dans le traitement ou la prévention des maladies liées à l'instabilité de l'ADN mitochondrial.

Plus particulièrement, l'invention concerne un composé de formule (a), pour une administration par voie intraveineuse à une dose de 20 à 150 µg/kg ou de 150 à 250 µg/kg, encore plus particulièrement à une dose de 20 à 50 µg/kg, de 50 à 100 µg/kg, de 100 à 150 µg/kg, de 150 à 200 µg/kg, ou de 200 à 250 µg/kg, pour son utilisation dans le traitement ou la prévention des maladies liées à l'instabilité de l'ADN mitochondrial.

Dans un autre mode de réalisation particulier, l'invention concerne un composé de formule (a) : 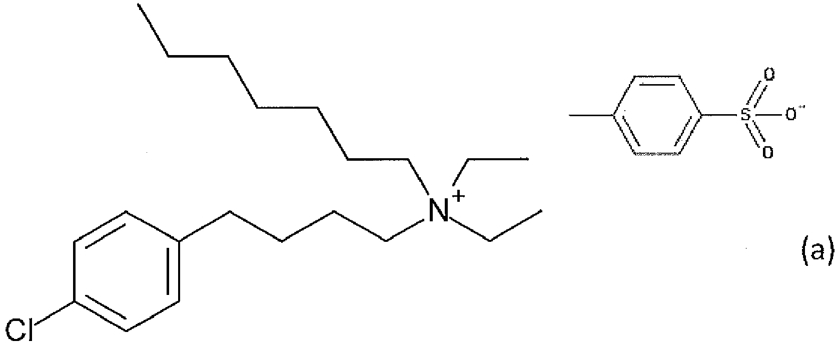 se présentant sous forme de dose unitaire de 0,5 mg à 30 mg, pour une administration par voie intraveineuse, pour son utilisation dans le traitement ou la prévention des maladies liées à l'instabilité de l'ADN mitochondrial.

Plus particulièrement, l'invention concerne un composé de formule (a), se présentant sous forme de dose unitaire de 0,5 mg à 12 mg ou de 12 à 30 mg, encore plus particulièrement se présentant sous forme de dose unitaire de 0,5 à 4 mg, de 4 à 8 mg, de 8 à 12 mg, de 12 à 16 mg, de 16 à 30 mg, de 16 à 20 mg ou de 20 à 30 mg, pour une administration par voie intraveineuse, pour son utilisation dans le traitement ou la prévention des maladies liées à l'instabilité de l'ADN mitochondrial.

Dans un autre mode de réalisation particulier, l'invention concerne un composé de formule (a) : 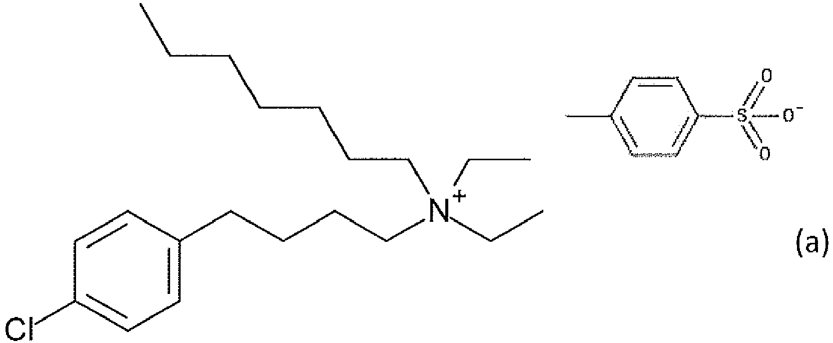 pour une administration par voie orale à une dose de 5 à 50 µg/kg, pour son utilisation dans le traitement ou la prévention des maladies liées à l'instabilité de l'ADN mitochondrial.

Plus particulièrement, l'invention concerne un composé de formule (a), pour une administration par voie orale à une dose de 5 à 30 µg/kg, ou de 30 à 50 µg/kg, encore plus particulièrement à une dose de 5 à 10 µg/kg, de 10 à 20 µg/kg, de 20 à 30 µg/kg, de 30 à 40 µg/kg ou de 40 à 50 µg/kg, pour son utilisation dans le traitement ou la prévention des maladies liées à l'instabilité de l'ADN mitochondrial.

Dans un autre mode de réalisation particulier, l'invention concerne un composé de formule (a), se présentant sous forme de dose unitaire de 150 µg à 6 mg, pour une administration par voie orale, pour son utilisation dans le traitement ou la prévention des maladies liées à l'instabilité de l'ADN mitochondrial.

Plus particulièrement, l'invention concerne un composé de formule (a), se présentant sous forme de dose unitaire de 150 µg à 2,5 mg ou de 2,5 à 6 mg, encore plus particulièrement de 150 µg à 800 µg, de 800 µg à 1,5 mg, de 1,5 à 2,5 mg, de 2,5 à 3,5 mg, de 3,5 à 6 mg, de 3,5 à 4,5 mg, ou de 4,5 à 6 mg, pour une administration par voie orale, pour son utilisation dans le traitement ou la prévention des maladies liées à l'instabilité de l'ADN mitochondrial.

Dans un autre mode de réalisation particulier, l'invention concerne un composé de formule (a), pour une administration par voie intraveineuse à une dose de 2 à 25 µg/kg, pour son utilisation dans le traitement ou la prévention des maladies liées à l'instabilité de l'ADN mitochondrial.

Plus particulièrement, l'invention concerne un composé de formule (a), pour une administration par voie intraveineuse à une dose de 2 à 15 µg/kg ou de 15 à 25 µg/kg, encore plus particulièrement à une dose de 2 à 5 µg/kg, de 5 à 10 µg/kg, de 10 à 15 µg/kg, de 15 à 20 µg/kg, ou de 20 à 25 µg/kg, pour son utilisation dans le traitement ou la prévention des maladies liées à l'instabilité de l'ADN mitochondrial.

Dans un autre mode de réalisation particulier, l'invention concerne un composé de formule (a), se présentant sous forme de dose unitaire de 50 µg à 3 mg, pour une administration par voie intraveineuse, pour son utilisation dans le traitement ou la prévention des maladies liées à l'instabilité de l'ADN mitochondrial.

Plus particulièrement, l'invention concerne un composé de formule (a), se présentant sous forme de dose unitaire de 50 µg à 1,2 mg ou de 1,2 à 3 mg, encore plus particulièrement se présentant sous forme de dose unitaire de 50 µg à 400 µg, de 400 à 800 µg, de 800 µg à 1,2 mg, de 1,2 à 1,6 mg, de 1,6 à 3 mg, de 1,6 à 2 mg ou de 2 à 3 mg, pour une administration par voie intraveineuse, pour son utilisation dans le traitement ou la prévention des maladies liées à l'instabilité de l'ADN mitochondrial.

Dans un autre mode de réalisation particulier, l'invention concerne un composé de formule (b) : 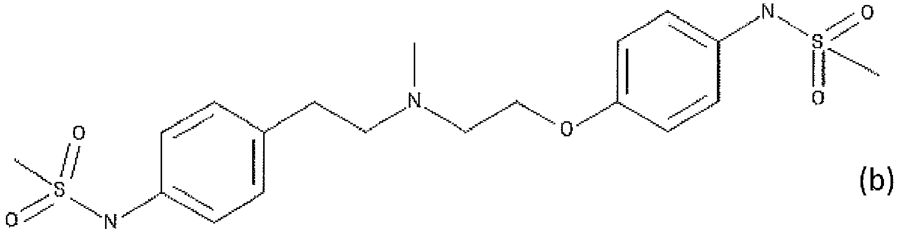 se présentant sous forme de dose unitaire de 125 à 500 µg deux fois par jour, pour une administration par voie orale, pour son utilisation dans le traitement ou la prévention des maladies liées à l'instabilité de l'ADN mitochondrial.

Plus particulièrement, l'invention concerne un composé de formule (b), se présentant sous forme de dose unitaire de 125 à 300 µg ou de 300 à 500 µg deux fois par jour, encore plus particulièrement se présentant sous forme de dose unitaire de 125 à 200 µg, de 200 à 300 µg, de 300 à 400 µg, ou de 400 à 500 µg deux fois par jour, pour une administration par voie orale, pour son utilisation dans le traitement ou la prévention des maladies liées à l'instabilité de l'ADN mitochondrial.

Dans un autre mode de réalisation particulier, l'invention concerne un composé de formule (b) : 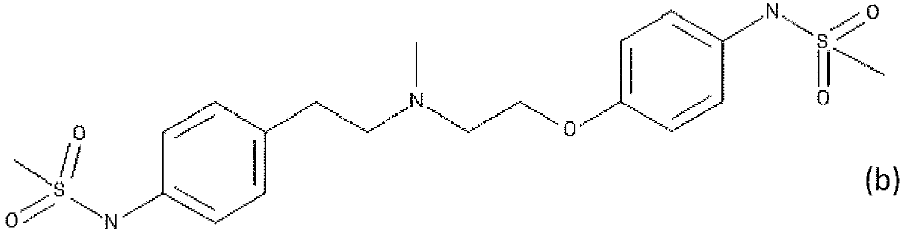 se présentant sous forme de dose unitaire de 12,5 à 50 µg, pour une administration par voie orale, pour son utilisation dans le traitement ou la prévention des maladies liées à l'instabilité de l'ADN mitochondrial.

Plus particulièrement, l'invention concerne un composé de formule (b), se présentant sous forme de dose unitaire de 12,5 à 30 µg ou de 30 à 50 µg, encore plus particulièrement se présentant sous forme de dose unitaire de 12,5 à 20 µg, de 20 à 30 µg, de 30 à 40 µg, ou de 40 à 50 µg, pour une administration par voie orale, pour son utilisation dans le traitement ou la prévention des maladies liées à l'instabilité de l'ADN mitochondrial.

Dans un autre mode de réalisation particulier, l'invention concerne un composé de formule (c) : 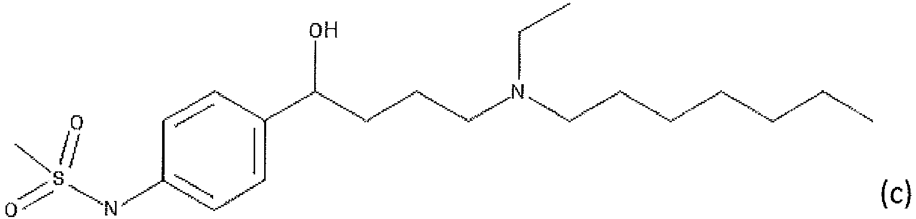 pour une administration par voie intraveineuse à une dose de 5 µg/kg à 20 µg/kg, pour son utilisation dans le traitement ou la prévention des maladies liées à l'instabilité de l'ADN mitochondrial.

Plus particulièrement, l'invention concerne un composé de formule (c), pour une administration par voie intraveineuse à une dose de 5 à 12 µg/kg ou de 12 à 20 µg/kg, encore plus particulièrement à une dose de 5 à 8 µg/kg, de 8 à 12 µg/kg, de 12 à 16 µg/kg, ou de 16 à 20 µg/kg, pour son utilisation dans le traitement ou la prévention des maladies liées à l'instabilité de l'ADN mitochondrial.

Dans un autre mode de réalisation particulier, l'invention concerne un composé de formule (c) : 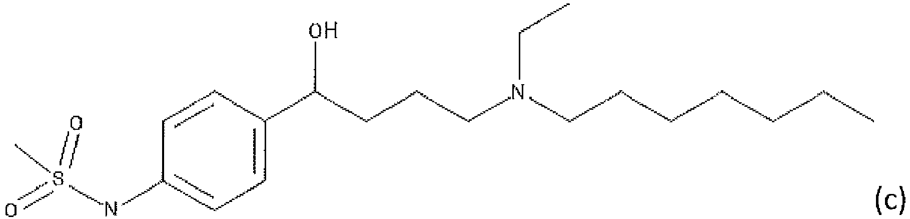 se présentant sous forme de dose unitaire de 150 µg à 2500 µg, pour une administration par voie intraveineuse, pour son utilisation dans le traitement ou la prévention des maladies liées à l'instabilité de l'ADN mitochondrial.

Plus particulièrement, l'invention concerne un composé de formule (c), se présentant sous forme de dose unitaire de 150 à 1000 µg ou de 1000 à 2500 µg, encore plus particulièrement se présentant sous forme de dose unitaire de 150 à 650 µg, de 650 à 1000 µg, de 1000 à 1500 µg, de 1500 à 2500 µg, de 1500 à 2000 µg, ou de 2000 à 2500 µg, pour une administration par voie intraveineuse, pour son utilisation dans le traitement ou la prévention des maladies liées à l'instabilité de l'ADN mitochondrial.

Dans un autre mode de réalisation, l'invention concerne un composé de formule (c) : 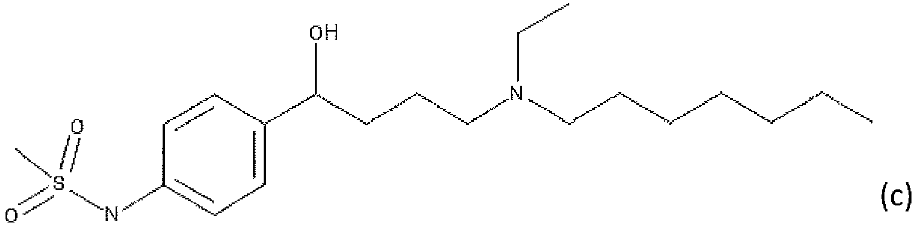 pour une administration par voie intraveineuse à une dose de 0,5 µg/kg à 2 µg/kg, pour son utilisation dans le traitement ou la prévention des maladies liées à l'instabilité de l'ADN mitochondrial.

Plus particulièrement, l'invention concerne un composé de formule (c), pour une administration par voie intraveineuse à une dose de 0,5 à 1,2 µg/kg ou de 1,2 à 2 µg/kg, encore plus particulièrement à une dose de 0,5 à 0,8 µg/kg, de 0,8 à 1,2 µg/kg, de 1,2 à 1,6 µg/kg, ou de 1,6 à 2 µg/kg, pour son utilisation dans le traitement ou la prévention des maladies liées à l'instabilité de l'ADN mitochondrial.

Dans un autre mode de réalisation particulier, l'invention concerne un composé de formule (c), se présentant sous forme de dose unitaire de 15 µg à 250 µg, pour une administration par voie intraveineuse, pour son utilisation dans le traitement ou la prévention des maladies liées à l'instabilité de l'ADN mitochondrial.

Plus particulièrement, l'invention concerne un composé de formule (c), se présentant sous forme de dose unitaire de 15 à 100 µg ou de 100 à 250 µg, encore plus particulièrement se présentant sous forme de dose unitaire de 15 à 65 µg, de 65 à 100 µg, de 100 à 150 µg, de 150 à 250 µg, de 150 à 200 µg, ou de 200 à 250 µg, pour une administration par voie intraveineuse, pour son utilisation dans le traitement ou la prévention des maladies liées à l'instabilité de l'ADN mitochondrial.

Avantageusement, l'invention concerne un composé de formule (a), (b) ou (c), se présentant sous forme de solution injectable, sirop, suspension, poudre, gélule, comprimé, pastille, granule, pilule ou suppositoire, pour son utilisation dans le traitement ou la prévention des maladies liées à l'instabilité de l'ADN mitochondrial.

Dans un mode de réalisation particulier, l'invention concerne un composé de formule (a), (b) ou (c), pour une administration par voie orale, se présentant sous forme de sirop, suspension, poudre, gélule, comprimé, pastille, granule ou pilule, pour son utilisation dans le traitement ou la prévention des maladies liées à l'instabilité de l'ADN mitochondrial.

Avantageusement, l'invention concerne un composé tel que défini ci-dessus, pour son utilisation dans le traitement ou la prévention des maladies liées à au moins une mutation, ou à au moins une délétion ou à au moins une insertion, ou à une combinaison de ces éléments, dans le gène POLG.

Dans un autre mode de réalisation, l'invention concerne un composé tel que défini ci-dessus, pour son utilisation dans le traitement ou la prévention des maladies liées à l'instabilité de l'ADN mitochondrial non liées aux mutations du gène POLG.

Dans un mode de réalisation particulier, l'invention concerne un composé tel que défini ci-dessus, pour son utilisation dans le traitement ou la prévention des maladies liées à une déplétion ou une délétion de l'ADN mitochondrial.

Dans un autre mode de réalisation particulier, l'invention concerne un composé tel que défini ci-dessus, pour son utilisation dans le traitement ou la prévention des maladies liées à une mutation ponctuelle de l'ADN mitochondrial.

Dans un mode de réalisation encore plus particulier, l'invention concerne un composé tel que défini ci-dessus, pour son utilisation dans le traitement ou la prévention
- de maladies associées à des anomalies quantitatives ou qualitatives de l'ADN mitochondrial telles que la maladie d'Alpers (AHS), le spectre des myo-cérébro-hépatopathies de l'enfant (MCHS), les épilepsies myocloniques avec myopathie et ataxie sensitive MEMSA (Myoclonic epilepsy myopathy sensory ataxia), l'ataxie spinocérébelleuse avec épilepsie (SCAE), les ataxies avec neuropathies incluant les syndromes MIRAS et SANDO, l'ophtalmoplégie externe progressive autosomique récessive (arPEO), l'ophtalmoplégie externe progressive autosomique dominante (adPEO), l'encéphalomyopathie mitochondriale neurogastrointestinale (MNGIE), le syndrome de Pearson, le syndrome de Kearns-Sayre, la myopathie infantile et l'amyotrophie spinale liées au mutations de *TK2*, les insuffisances hépatiques avec déplétion de l'ADNmt, les pathologies associées aux mutations des gènes *SUCLA2* et *SUCLAG1*, *RRM2B*, *AIF1*, *MPV17*, ou
- des maladies associées à des mutations ponctuelles de l'ADN mitochondrial telles que la neuropathie optique héréditaire de Leber, du syndrome MELAS, du syndrome MERRF, et certaines formes de syndrome de Leigh, d'ophtalmoplégie externe progressive chronique, de myopathie, de cardiomyopathie, de diabète-surdité, d'encéphalomyopathie et de surdité.

Dans un mode de réalisation particulier, l'invention concerne un composé tel que défini ci-dessus, pour son utilisation dans le traitement ou la prévention des maladies liées à au moins une mutation, ou à au moins une délétion ou à au moins une insertion, ou à une combinaison de ces éléments, dans le gène POLG, lesdites maladies comprenant la maladie d'Alpers (AHS), le spectre des myo-cérébro-hépatopathies de l'enfant (MCHS), les épilepsies myocloniques avec myopathie et ataxie sensitive MEMSA (Myoclonic epilepsy myopathy sensoryataxia), les ataxies spinocérébelleuses avec épilepsie (SCAE), les ataxies avec neuropathies incluant les syndromes MIRAS et SANDO, l'ophialmoplégie externe progressive autosomique récessive (arPEO) et l'ophtalmoplégie externe progressive autosomique dominante (adPEO).

Dans un autre mode de réalisation particulier, l'invention concerne un composé tel que défini ci-dessus, pour son utilisation dans le traitement ou la prévention des maladies liées à une déplétion ou une délétion de l'ADN mitochondrial, lesdites maladies comprenant l'ophtalmoplégie externe progressive autosomique récessive, l'encéphalomyopathie mitochondriale neurogastrointestinale, le syndrome de Pearson, le syndrome de Kearns-Sayre, la myopathie infantile et l'amyotrophie spinale liées aux mutations de *TK2*, les insuffisances hépatiques avec déplétion de l'ADNmt, les pathologies associées aux mutations des gènes *SUCLA2* et *SUCLAG1*, *RRM2B*, *AIF1*, *MPV17* et l'ophtalmoplégie externe progressive autosomique dominante.

Dans un autre mode de réalisation particulier, l'invention concerne un composé tel que défini ci-dessus, pour son utilisation dans le traitement ou la prévention des maladies liées à une mutation ponctuelle de l'ADN mitochondrial, lesdites maladies comprenant la neuropathie optique de Leber, le syndrome MELAS, le syndrome MERRF, et certaines formes de syndrome de Leigh, d'ophtalmoplégie externe progressive chronique, de myopathie, de cardiomyopathie, de diabète-surdité, d'encéphalomyopathie et de surdité.

Dans un mode de réalisation encore plus particulier, l'invention concerne un composé tel que défini ci-dessus, pour son utilisation dans le traitement ou la prévention du syndrome MELAS.

Le syndrome MELAS (Mitochondrial Encephalopathy Lactic Acidosis Stroke) est une mitochondropathie due le plus fréquemment à une mutation A3243G dans l'ARNt^{Leu} de l'ADN mitochondrial. La maladie débute le plus souvent dans l'enfance ou chez les jeunes adultes. Chez les sujets atteints de ce syndrome, il existe souvent des symptômes chroniques comme une myocardiopathie, une surdité, un diabète, une petite taille, une faiblesse musculaire, un retard mental, des troubles de l'apprentissage, de la mémoire ou de l'attention. Pour les formes MELAS les plus sévères, des accidents vasculaires à répétition sont également responsables d'atteintes neurologiques graves. Les patients atteints du syndrome MELAS présentent une hyperlactacidémie dans leur sang, c'est-à-dire une augmentation du lactate, dû au dysfonctionnement mitochondrial et au déficit énergétique qui en découle. On observe également chez les individus atteints du syndrome MELAS une réduction significative de l'activité du complexe I, qui est la première enzyme de la chaine respiratoire mitochondriale. Il est ainsi attendu qu'un médicament efficace pour le traitement du syndrome MELAS augmente l'activité du complexe I et réduise la production de lactate (par augmentation de la production énergétique) chez le patient.

### DESCRIPTION DES FIGURES

**Figure 1** **:** La figure 1 représente la densité optique (DO₆₀₀) d'une culture cellulaire de levure *Saccharomyces cerevisiae* mutante dans le gène *MIP1* (*mip1^{G651S}*) après 24 heures de culture en fonction de la concentration de clofilium tosylate ajoutée au milieu de culture.
**Figure 2** **:** La figure 2 représente la fréquence, en pourcentage, de production de colonies *petite* de la souche de levure *mip1*^{G651S} en présence (CLOF) et absence (DMSO) de clofilium tosylate 32 µM dans le milieu de culture.
**Figure 3** : La partie A représente la fréquence, en pourcentage, de production de colonies *petite* des mutants *mip1* récessifs.
   La partie B représente la réduction du nombre de colonies *petite* en présence de clofilium tosylate 32 µM comparée au nombre de cellules non traitées (cellules en présence de DMSO) chez les mutants *mip1* récessifs.
   La partie C représente la fréquence, en pourcentage, de production de colonies *petite* des mutants *mip1* dominants.
   La partie D représente la réduction du nombre de colonies *petite* en présence de clofilium tosylate 32 µM comparée au nombre de cellules non traitées (cellules en présence de DMSO) chez les mutants *mip1* dominants.

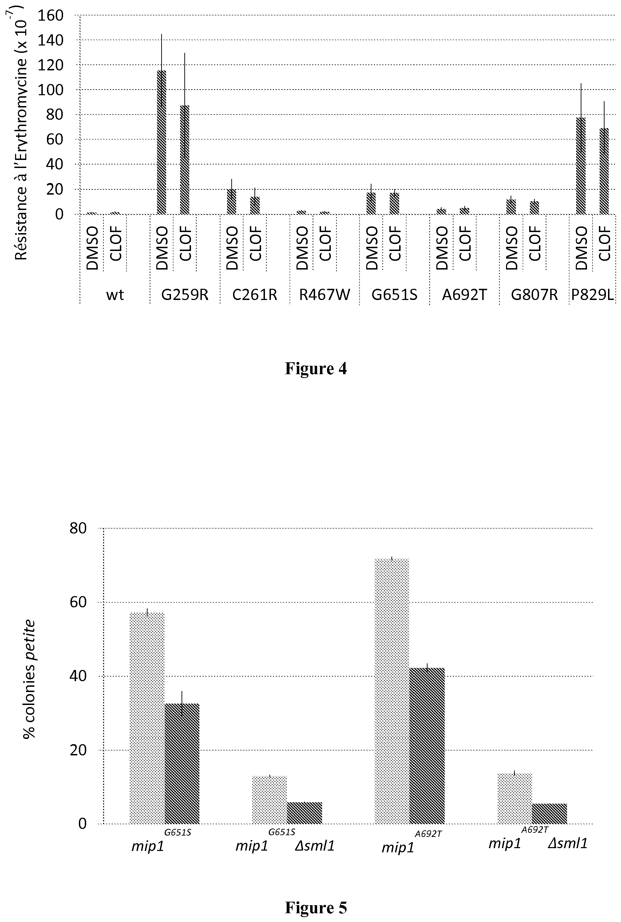

**Figure 4** **:** La figure 4 représente le nombre de colonies résistantes à l'érythromycine (Ery^{R}) sur le nombre total de colonies (fréquence des cellules résistantes à l'érythromycine) des mutants *mip1* récessifs en absence (DMSO) et en présence (CLOF) de clofilium tosylate 32 µM.
**Figure 5** **:** La figure 5 représente la fréquence, en pourcentage, de production de colonies *petite,* chez les souches de levures *mip1^{G651S}*, *mip1^{G651S} Δsml1*, *mip1^{A692T}* et *mip1^{A692T}Δsml1,* en présence de clofilium tosylate 32 µM (gris foncé) ou en absence de clofilium tosylate (présence de DMSO, gris clair).

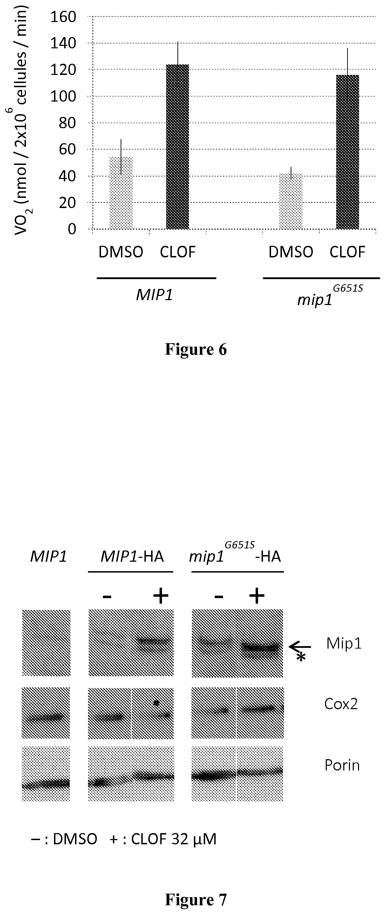

**Figure 6** **:** La figure 6 représente la vitesse de consommation de dioxygène (VO₂) en nmol/2x10⁶ cellules/min chez des levures exprimant le gène sauvage *MIP1* ou le gène muté *mip1^{G651S}*, en présence (CLOF) ou en absence (DMSO) de clofilium tosylate 32 µM.
**Figure 7** **:** La figure 7 représente l'analyse par électrophorèse sur gel SDS/PAGE des fractions protéiques mitochondriales des cellules de levure cultivées en présence (CLOF, +) ou en absence (DMSO, -) de clofilium tosylate 32 µM. Les protéines étudiées sont la protéine Mip1-HA, la protéine Mip1G651S-HA (analyse sur gel SDS/PAGE 6%), la protéine mitochondriale Cox2 et la porine (analyse sur gel SDS/PAGE 10%). Les protéines après séparation sont transférées sur membrane de nitrocellulose puis révélées par détection de l'activité peroxydase ECL^{Plus} à l'aide d'anticorps primaires et d'anticorps secondaires couplés à la horseradish peroxydase (HRP). La bande marquée d'une * est une réaction non spécifique de l'anticorps anti-HA.

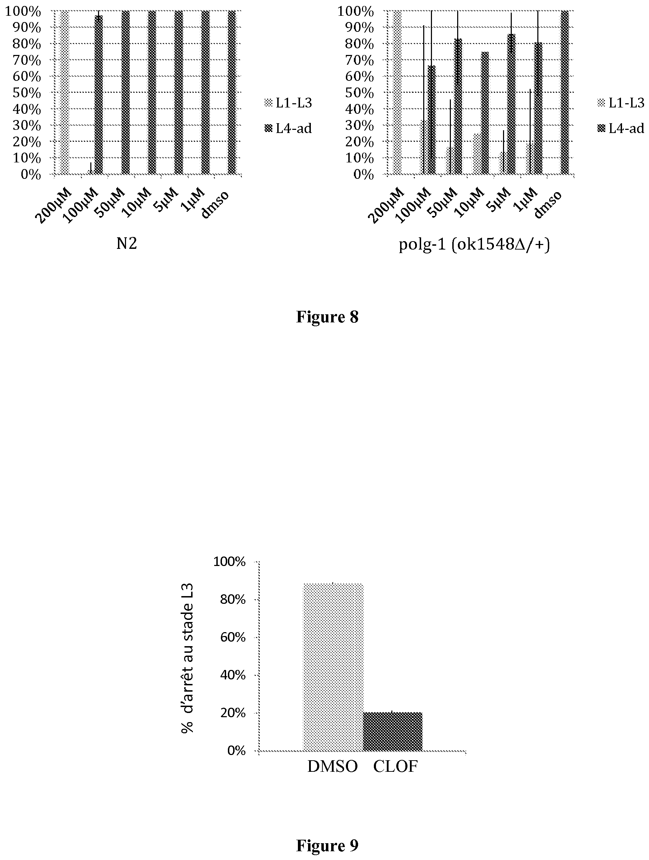

**Figure 8** **:** La figure 8 représente le pourcentage de nématode *Caenorhabditis elegans* aux stades L1 à L3 et au stade L4-Adulte par rapport au nombre total de nématodes chez une lignée sauvage (N2) et chez une lignée mutée TB2143 (polg-1(ok1548Δ/+)) portant une délétion hétérozygote de 2149 bp dans la séquence codante du gène polg-1, en fonction de la concentration en clofilium tosylate contenue dans le milieu de culture des nématodes (boîtes NGM).
**Figure 9** **:** La figure 9 représente le pourcentage de larves de nématode TB2143 (polg-1(ok1548Δ/+)) haploinsuffisants élevées sur des boîtes NGM contenant 30 µg/mL de bromure d'éthidium dont le développement s'est arrêté au stade L3 selon l'absence (DMSO) ou la présence (CLOF) de clofilium tosylate 50 µM dans les boîtes.

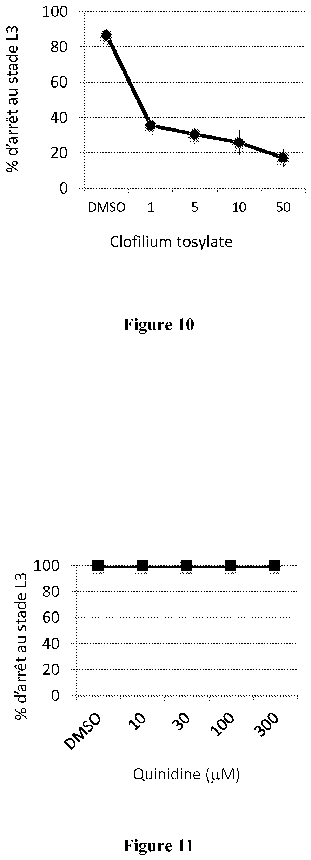

**Figure 10** **:** La figure 10 représente la courbe dose-réponse du clofilium tosylate sur la résistance des nématodes TB2143 haploinsuffisants (polg-1(ok1548Δ/+)) au bromure d'éthidium : le pourcentage de larves L3 (arrêt du développement) est représenté en fonction de la concentration de clofilium tosylate contenue dans les boîtes NGM des nématodes comprenant 30 µg/mL de bromure d'éthidium.
**Figure 11** : La figure 11 représente la courbe dose-réponse de la quinidine sur la résistance des nématodes TB2143 haploinsuffisants (polg-1(ok1548Δ/+)) au bromure d'éthidium : le pourcentage de larves L3 (arrêt du développement) est représenté en fonction de la concentration de quinidine contenue dans les boîtes NGM des nématodes comprenant 30 µg/mL de bromure d'éthidium.

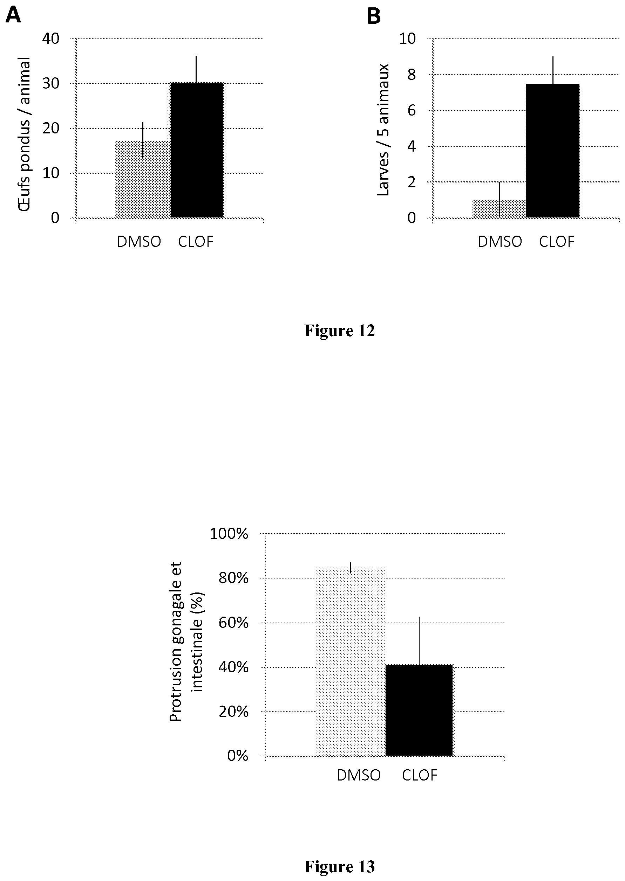

**Figure 12** : La partie A représente le nombre d'oeufs pondus par des nématodes homozygotes TB2143 (polg-1(Δ/Δ)) en absence (DMSO) ou en présence (CLOF) de clofilium tosylate 50 µM dans les boîtes NGM des nématodes.
   La partie B représente le nombre de larves, donc d'éclosions des oeufs pondus, par 5 nématodes homozygotes TB2143 (polg-1(Δ/Δ)) en absence (DMSO) ou en présence (CLOF) de clofilium tosylate 50 µM dans les boîtes NGM des nématodes.
**Figure 13** : La figure 13 représente le pourcentage de nématodes TB2143 homozygotes (polg-1(ok1548Δ/Δ)) morts et présentant une extrusion de l'intestin et/ou des gonades au 7^{ème} jour de l'adulte en absence (DMSO) et en présence (CLOF) de clofilium tosylate 50 µM.

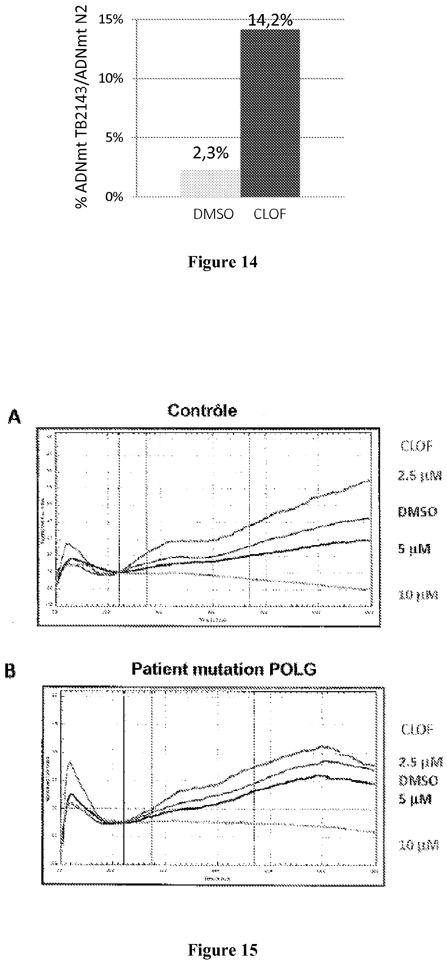

**Figure 14** **:** La figure 14 représente le pourcentage d'ADNmt des nématodes TB2143 homozygotes (polg-1(ok1548Δ/Δ)) par rapport à la souche de référence N2 en présence de DMSO et en présence de clofilium tosylate 50 µM.
**Figure 15** : La figure 15 représente l'index cellulaire, représentatif de la viabilité des cellules, de fibroblastes humains cultivés avec différentes concentrations de clofilium tosylate en fonction du temps.
   La partie A représente l'index cellulaire des fibroblastes contrôles.
   La partie B représente l'index cellulaire de fibroblastes d'un patient ayant une mutation sur chaque allèle du gène POLG.

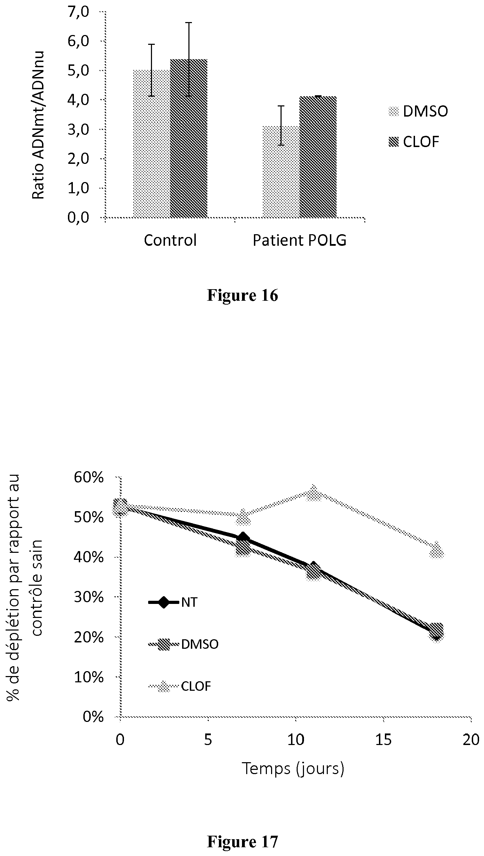

**Figure 16** **:** La figure 16 représente le ratio de la quantité d'ADNmt sur la quantité d'ADN nucléaire (ADNnu) chez les fibroblastes contrôles et les fibroblastes d'un patient présentant une mutation sur chaque allèle du gène POLG cultivés en absence (DMSO) et en présence (CLOF) de clofilium tosylate 2,5 µM.
**Figure 17** **:** La figure 17 représente le pourcentage de déplétion de l'ADNmt de fibroblastes cutanés d'un patient présentant une mutation sur chaque allèle du gène POLG comparé à la quantité d'ADNmt de fibroblastes cutanés contrôles, cultivés en milieu quiescent en absence de tout traitement (NT) ou en présence de DMSO (DMSO) ou présence de clofilium tosylate 1 µM (CLOF) en fonction du temps.

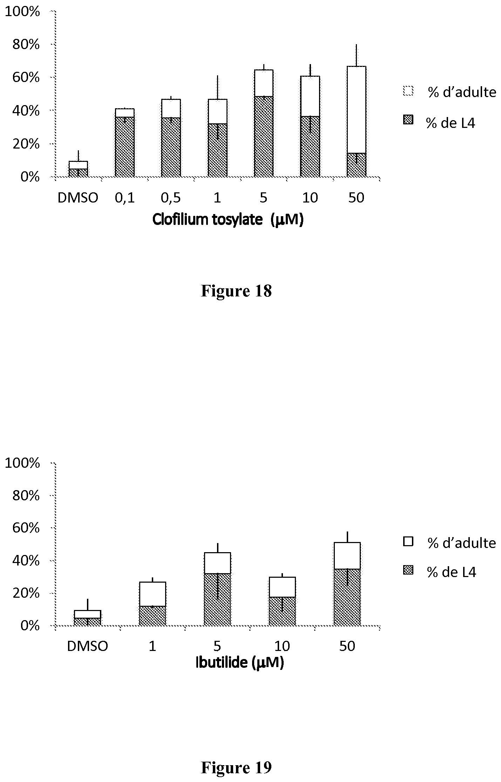

**Figure 18** **:** La figure 18 représente le pourcentage de nématodes haploinsuffisants TB2143 (polg-1(ok1548Δ/+)) au stade adulte et au stade de développement L4 après 4 jours d'élevage sur des boîtes NGM supplémentées avec 30 µg/mL de bromure d'éthidium et différentes concentrations de clofilium tosylate ou du DMSO.
**Figure 19** **:** La figure 19 représente le pourcentage de nématodes haploinsuffisants TB2143 (polg-1(ok1548Δ1+)) au stade adulte et au stade de développement L4 après 4 jours d'élevage sur des boîtes NGM supplémentées avec 30 µg/mL de bromure d'éthidium et différentes concentrations d'Ibutilide hemifuramate ou du DMSO.

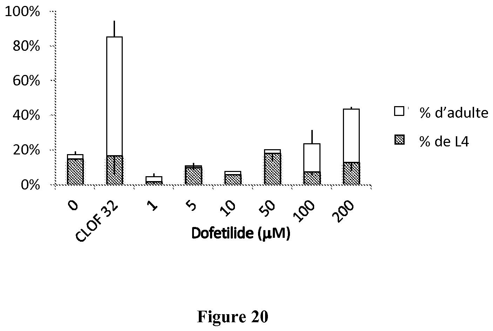

**Figure 20** : La figure 20 représente le pourcentage de nématodes haploinsuffisants TB2143 (polg-1(ok1548Δ/+)) au stade adulte et au stade de développement L4 après 4 jours d'élevage sur des boîtes NGM supplémentées avec 30 µg/mL de bromure d'éthidium et du clofilium tosylate 50 µM, ou différentes concentrations de Dofétilide ou du DMSO.

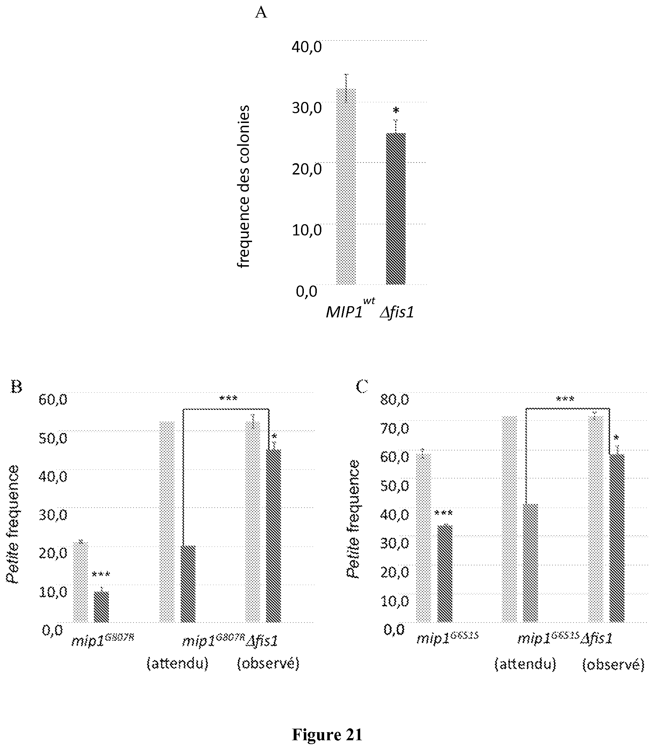

**Figure 21** : La partie A représente la fréquence du nombre de colonies *petite* en présence de clofilium tosylate 32 µM (gris foncé) comparée à la fréquence du nombre de colonies *petite* de cellules non traitées (DMSO, gris clair) chez le mutant *Δfis1*.
   La partie B représente la fréquence du nombre de colonies *petite* en présence de clofilium tosylate 32 µM (gris foncé) comparée à la fréquence du nombre de colonies *petite* de cellules non traitées (DMSO, gris clair) chez le mutant *mip1^{G807R}* ainsi que la fréquence calculée (attendu) et celle observée chez le double mutant *mip1^{G807R}Δfis1*.
   La partie C représente la fréquence du nombre de colonies *petite* en présence de clofilium tosylate 32 µM (gris foncé) comparée à la fréquence du nombre de colonies *petite* de cellules non traitées (DMSO, gris clair) chez le mutant *mip1^{G651S}* ainsi que la fréquence calculée (attendu) et celle observée chez le double mutant *mip1^{G651S}Δfis1.*

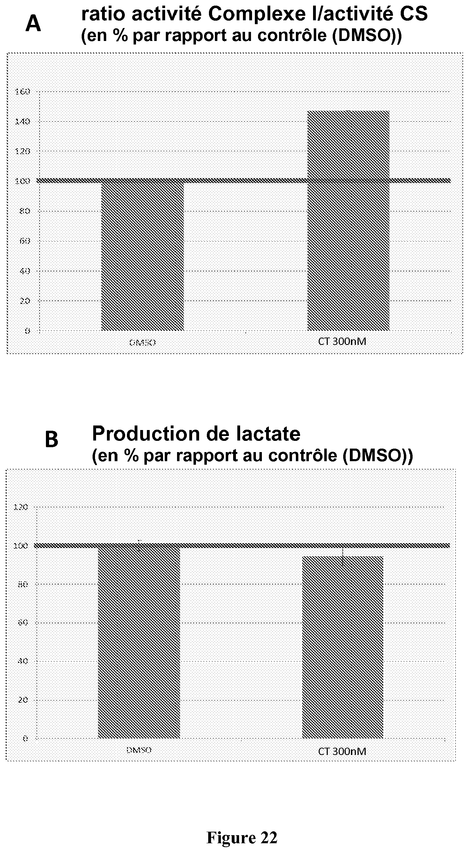

**Figure 22** : La figure 22 représente l'effet du clofilium tosylate sur le ratio des activités du complexe I / citrate synthase et la production de lactate de cybrides neuronaux dérivés d'un patient MELAS (m.3243A>G tRNA^{leu} mutation).
   La partie A représente le ratio des activités complexe I /citrate synthase, en pourcentage par rapport au contrôle, après traitement des cybrides neuronaux avec du DMSO (contrôle) ou 300 nM (CT 300nM) de clofilium tosylate, après 48 heures de traitement. N= 4. CS = citrate synthase.
   La partie B représente la production de lactate, en pourcentage par rapport au contrôle, par les cybrides neuronaux après traitement des cybrides neuronaux avec du DMSO (contrôle) ou 300 nM (CT 300nM) de clofilium tosylate pendant 48 heures. N=4.

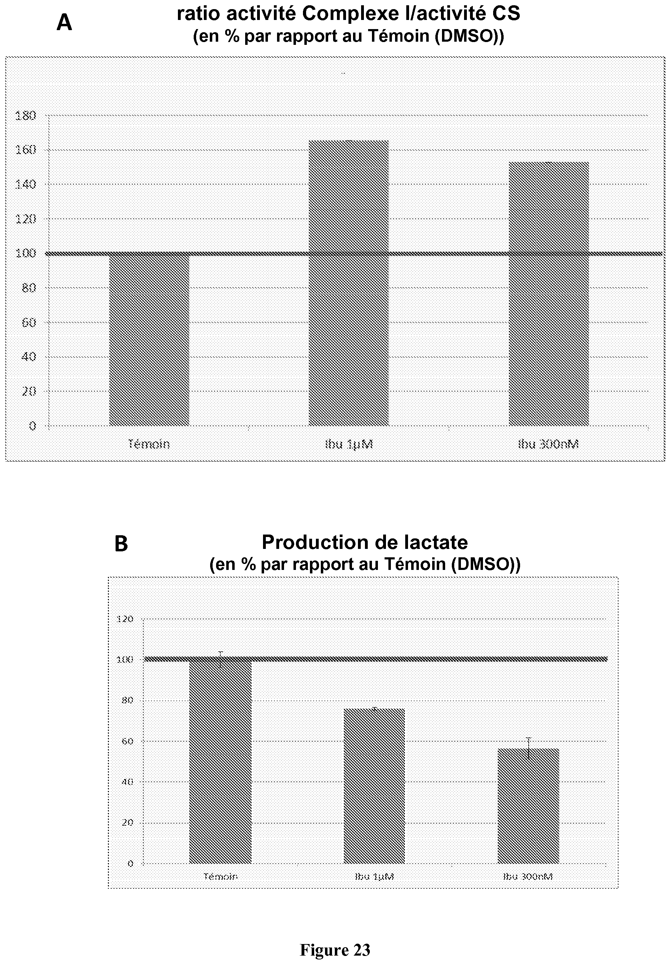

**Figure 23** : La figure 23 représente l'effet dose de l'ibutilide sur le ratio des activités du complexe I/citrate synthase et la production de lactate des cybrides neuronaux dérivés d'un patient MELAS (m.3243A>G tRNA^{leu} mutation).
   La partie A représente le ratio des activités complexe I /citrate synthase, en pourcentage par rapport au témoin, après traitement des cybrides neuronaux avec du DMSO (Témoin), ou 1 µM (ibu 1µM) ou 300 nM (ibu 300 nM) d'ibutilide pendant 48 heures. N=4. CS = citrate synthase. La partie B représente la production de lactate, en pourcentage par rapport au témoin, par les cybrides neuronaux après traitement des cybrides neuronaux avec du DMSO (Témoin) ou 1 µM (ibu 1µM) ou 300 nM (ibu 300nM) d'ibutilide pendant 48 heures. N= 4.

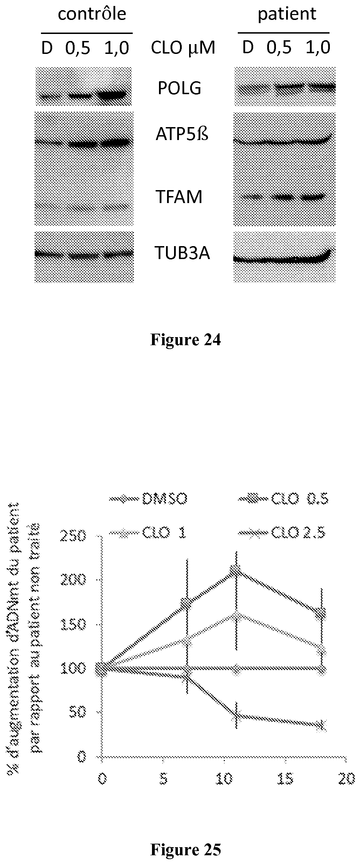

**Figure 24** **:** La figure 24 représente l'analyse par électrophorèse sur gel SDS/PAGE des fractions protéiques d'extraits totaux de fibroblastes contrôles (contrôle) et de fibroblastes d'un patient avec une mutation sur chaque allèle du gène POLG (patient) en condition de prolifération cellulaire. Les fibroblastes sont cultivés en présence de DMSO 0,1% (D) ou avec 0,5 µM ou 1,0 µM de clofilium tosylate (CLO). Les protéines étudiées sont la protéine POLG (analyse sur gel SDS/PAGE 6%), les protéines mitochondriales ATP5B et TFAM ainsi que la protéine cytosolique TUB3A comme contrôle de charge.
**Figure 25** : La figure 25 représente le pourcentage d'augmentation du nombre de copies d'ADNmt de fibroblastes cutanés d'un patient présentant une mutation sur chaque allèle du gène POLG comparé au nombre de copies d'ADNmt des fibroblastes du patient non traités (DMSO), cultivés en milieu quiescent en présence de DMSO (DMSO) ou en présence de différentes concentrations de clofilium tosylate : 0,5 µM (CLO 0.5), 1 µM (CLO 1), 2,5 µM (CLO 2.5), en fonction du nombre de jours de traitement (traitement de 18 jours).

### EXEMPLES

Les exemples 1 à 14, 17 à 22 et 24 à 25 concernent le clofilium tosylate: les exemples 1 à 6 et 17 concernent la levure *Saccharomyces cerevisiae*, les exemples 7 à 11 et 20 concernent le nématode *Caenorhabditis elegans,* les exemples 12 à 14, 18, 19, 24 et 25 concernent les fibroblastes humains et l'exemple 22 concerne les cybrides neuronaux dérivés d'un patient MELAS (m.3243A>G tRNA^{leu} mutation). L'exemple 21 concerne la combinaison du clofilium tosylate avec le resvératrol et les fibroblastes humains.

L'exemple 15 concerne l'Ibutilide et le nématode, l'exemple 23 concerne l'Ibutilide et les cybrides neuronaux dérivés d'un patient MELAS (m.3243A>G tRNA^{leu} mutation), l'exemple 16 concerne le Dofétilide et le nématode.

### Matériel et méthodes

Les levures *S*. *cerevisiae* utilisées dans les exemples 1 à 6 et 17 sont des levures de fond génétique DWM-5A : Mat a *ade2-1 leu2-3, 112 ura3-1 trp1-1 his3-11, 15 can1-100 Δmip1::KanR* transformées par un plasmide mono-copie (ARS-CEN) pFL39 (*TRP1*) permettant l'expression des différents allèles de *MIP1* (Baruffini, E., Lodi, T., Dallabona, C., Puglisi, A., Zeviani, M. and Ferrero, I. (2006) Genetic and Chemical rescue of the Saccharomyces cerevisiae phenotype induced by mitochondrial DNA polymerase mutations associated with progressive external ophtalmoplegia in humans. Hum. Mol. Genet., 15, 2846 - 2855).

Les nématodes *C*. *elegans* utilisés dans les exemples 7 à 11 et 15, 16 et 20 sont la lignée BRISTOL N2 sauvage, la lignée VC1224 (polg-1(ok1548)/mT1 II; +/mT1[dpy-10(eI28)] II) du consortium Caenorhabditis Genetics Center (CGC), la lignée TB2143 (polg-1 (ok1548)/+ II; +/ mln [dpy-10(e128) mls14] II) (A. Trifunovic, Allemagne) et la lignée DA631 (eat-3(ad426) II ; him-8(e1489) IV) (CGC).

### Exemple 1 : Détermination de la concentration maximale de clofilium tosylate tolérée par la souche de levure Saccharomyces cerevisiae mip1^{G651S}.

L'inhibition de croissance (MIC, Minimum Inhibitory Concentration) du clofilium tosylate sur la souche *mip1*^{G651S} est testée pour des concentrations de clofilium tosylate de 512 µM, 256 µM, 128 µM, 64 µM, 32 µM, 16 µM, 8 µM, 4µM, 2 µM, et 1 µM.

A partir d'une préculture o/n en milieu selectif (SC-URA, 2% éthanol (v/v)), 50 mL de milieu riche YPD (1% Yeast Extract, 0,5% Bacto Peptone, 2% Glucose) sont ensemencés à une DO₆₀₀ de 0,05/mL. Ces 50 mL de culture sont répartis dans une série de dix tubes, à raison de 2 mL de culture / tube, et 4 mL de culture sont déposés dans un dernier tube. Dans ce dernier tube (tube 1) est ajouté le clofilium tosylate à la concentration maximale : 512 µM (solubilité maximale). De ce tube 1, 2 mL de culture sont mis dans le tube 2. Le tube 2 contient 4 mL de culture et le clofilium tosylate à une concentration de 256 µM. 2 mL du tube 2 sont ajoutés au tube 3 et ainsi de suite. Chaque tube contient la drogue à une concentration égale à la moitié de celle du tube précédent. Deux contrôles sont effectués, sans clofilium tosylate et avec du DMSO (le volume étant le même que celui qui a servi à diluer le clofilium tosylate). Les cultures sont incubées à 28°C avec agitation pendant 24 heures et la densité optique (DO₆₀₀) est mésurée.

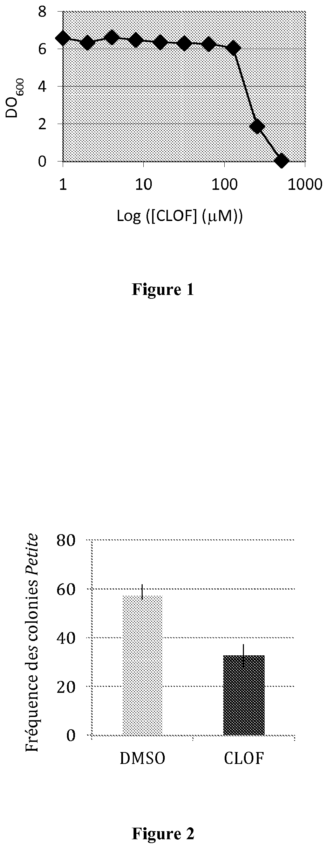

Les résultats sont présentés en Figure 1.

La concentration choisie de clofilium tosylate pour l'étude de son effet chez la levure S. *cerevisiae* est de 32 µM.

### Exemple 2 : Effet du clofilium tosylate sur la production de colonies petite chez des souches mip1 mutées de levure

Le taux de cellules *petite* dans une population représente les cellules ne respirant pas du fait de remaniements de l'ADNmt et/ou ayant perdu leur ADNmt.

### 2.1. Mode opératoire

A partir d'une préculture en milieu selectif (SC-TRP, 2% éthanol (v/v)) de la souche *mip1* mutée, 2.10⁵ cellules/mL sont ensemencées dans 2 mL de milieu riche YPD (1% Yeast Extract, 0,5% Bacto Peptone, 2% Glucose) en présence de clofilium tosylate 32 µM ou de DMSO. Puis les cellules sont incubées à 28°C pendant deux cycles de 24 heures. Les cellules sont ensuite diluées pour avoir environ 200 à 250 cellules par boîte et étalées sur milieu synthétique différentiel (SC-TRP, 0,3% glucose, 2% éthanol). Le nombre de colonies *petite* est calculé après 6 jours d'incubation à 28°C (Baruffini, E., Lodi, T., Dallabona, C., Puglisi, A., Zeviani, M. and Ferrero, I. (2006) Genetic and Chemical rescue of the Saccharomyces cerevisiae phenotype induced by mitochondrial DNA polymerase mutations associated with progressive external ophtalmoplegia in humans. Hum. Mol. Genet., 15, 2846 - 2855).

### 2.2. Effet du clofilium tosylate sur la production de colonies petite de la souche de levure mutante mip1^{G651S}

L'effet du clofilium tosylate sur la production de colonies *petite* est mesuré chez la levure mutante *mip1^{G651S}.*

Les résultats sont présentés en Figure 2.

Le clofilium tosylate réduit la production de colonies *petite* de la souche de levure mutante *mip1^{G651S}.*

### 2.3. Effet de clofilium tosylate sur la production de colonies petite sur différents mutants récessifs et dominants de mip1 chez la levure

L'effet du clofilium tosylate sur la production de colonies *petite* est mesuré chez 8 mutants récessifs *mip1* :
- H734Y (domaine muté: polymérase)
- G259R (domaine muté: exonuclease)
- C261R (domaine muté: exonuclease)
- P829L (domaine muté: polymérase)
- A692T (domaine muté: polymérase)
- G651S (domaine muté: polymérase)
- R467W (domaine muté: linker)
- G807R (domaine muté: polymérase)
et chez 3 mutants dominants *mip1* :
- Y757C (domaine muté: polymérase)
- K749R (domaine muté: polymérase)
- E698G (domaine muté: polymérase)

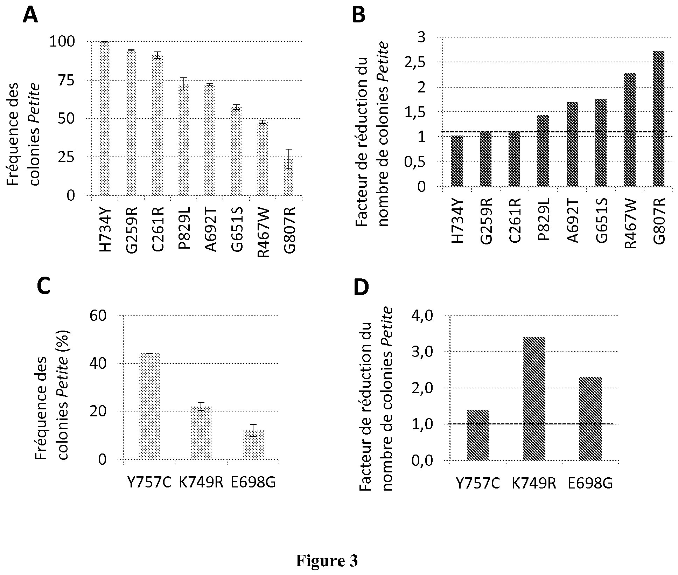

### Les résultats sont présentés en Figure 3.

Le clofilium tosylate réduit la production de cellules ne respirant pas quel que soit le domaine de *MIP1* muté (exonucléase, polymérase ou linker) à l'exception des mutations produisant plus de 90% de colonies *petite.*

### Exemple 3 : Effet du clofilium tosylate sur la fidélité des différents mutants de MIP1 chez la levure

Une perturbation de la fidélité de la polymérase mitochondriale Mip1 peut être évaluée en mesurant la fréquence des cellules résistantes à l'érythromycine (Ery^{R}). Les mutations Ery^{R} sont causées par des mutations dans l'ARN ribosomique mitochondrial 21S codé par l'ADNmt qui peuvent se produire après incorporation d'un mauvais nucléotide (Baruffini, E., Lodi, T., Dallabona, C., Puglisi, A., Zeviani, M. and Ferrero, I. (2006) Genetic and Chemical rescue of the Saccharomyces cerevisiae phenotype induced by mitochondrial DNA polymerase mutations associated with progressive external ophtalmoplegia in humans. Hum. Mol. Genet., 15, 2846 - 2855). Une mesure de la résistance à l'érythromycine est un moyen de mesurer directement les mutations ponctuelles de l'ADNmt.

Des colonies indépendantes sont ensemencées dans 10 mL de milieu minimum (SC-TRP, 2% glucose) en présence de clofilium tosylate 32 µM ou de DMSO jusqu'à la phase stationnaire. Pour déterminer le nombre total de cellules capables de respirer, un aliquot de chaque culture est étalé sur milieu riche YPE (1% Yeast Extract, 0,5% Bacto Peptone, 2% Ethanol). Le reste des cultures est étalé sur milieu N1 contenant 2,5 mg/mL d'erythromycine (2% peptone, 1% yeast extract, 40 mg/L adenine, 3% éthanol, 3 % glycerol dans 25 mM de tampon phosphate pH 6,5 et supplémenté avec 2,5 g/L d'erythromycine (SIGMA)) et incubé à 28°C pendant 8 jours. L'expérience est réalisée en duplicat. La fréquence des mutations est calculée comme le nombre de colonies Ery^{R} sur le nombre total de colonies (Baruffini, E., Lodi, T., Dallabona, C., Puglisi, A., Zeviani, M. and Ferrero, I. (2006) Genetic and Chemical rescue of the Saccharomyces cerevisiae phenotype induced by mitochondrial DNA polymerase mutations associated with progressive external ophtalmoplegia in humans. Hum. Mol. Genet., 15, 2846 - 2855).

Les résultats sont présentés en Figure 4.

Le clofilium tosylate n'a aucun effet sur la fidélité des différents mutants de levure de *MIP1.*

### Exemple 4 : Effet du clofilium tosylate et de la disponibilité des dNTP sur la stabilité de l'ADNmt des mutants de levure mip1^{G6S1S} et mip1^{A692T}

La délétion du gène *SML1* codant l'inhibiteur de *RNR1* (ribonucleotide-diphosphate reductase, enzyme limitante de la voie de synthèse des dNTP) est connue pour être un suppresseur génétique de l'instabilité de l'ADNmt due à des mutations dans le gène *MIP1* (Wang PJ, Chabes A, Casagrande R, Tian XC, Thelander L and Huffaker TC. (1997) Rnr4p a novel ribonucleotide reductase small-subunit protein. Mol Cell Biol, 17, 6114-6121).

La fréquence de colonies *petite* est déterminée pour les souches *mip1^{G651S}* et *mip1^{A692T}* délétées ou non du gène *SML1* et en présence de clofilium tosylate 32 µM ou de DMSO.

A partir de précultures en milieu selectif (SC-TRP, 2% éthanol (v/v)) des souches *mip1* mutées, 2.10⁵ cellules/mL sont ensemencées dans 2 mL de milieu riche YPD en présence de clofilium tosylate 32 µM ou de DMSO puis incubées à 28°C pendant 24 heures. Les cellules sont ensuite diluées pour avoir environ 200 à 250 cellules par boîte et étalées sur milieu synthétique différentiel (SC-TRP, 0,3% glucose, 2% éthanol). Le nombre de colonies *petite* est calculé après 5 jours d'incubation à 28°C.

### Les résultats sont présentés en Figure 5.

Le clofilium tosylate et la disponibilité des dNTP (due à la délétion du gène *SML1*) ont un effet additif sur la stabilité de l'ADNmt des mutants de levure *mip1^{G651S}* et *mip1^{A692T}.*

### Exemple 5 : Effet du clofilium tosylate sur la respiration des cellules de levure

L'intensité respiratoire est la quantité d'oxygène consommée par unité de temps et de matière biologique. Elle reflète l'activité du métabolisme oxydatif des cellules.

La consommation d'oxygène sur cellules entières est mesurée à l'aide d'une électrode HANSATECH. Les cellules de levure exprimant le gène sauvage *MIP1* ou le gène muté *mip1^{G651S}* sont cultivées pendant 7-8 générations dans un milieu YPE (1% Yeast Extract, 0,5% Bacto Peptone, 2% Ethanol) supplémenté avec du DMSO ou du clofilium tosylate 32 µM à 28°C avec agitation. Un volume correspondant à un total de 60 unités de DO₆₀₀ de chaque culture est centrifugé pendant 5 min à 3000 g. Le culot est resuspendu en YPE afin d'avoir 6.10⁵ cellules/µL. 50 µL, soit 3.10⁷ cellules, sont introduits dans la chambre de mesure de l'électrode HANSATECH qui est maintenue à 28°C. La consommation d'O₂ est observée et enregistrée en temps réel. La vitesse de consommation d'O₂ est calculée à partir de la partie linéaire de la courbe de consommation d'O₂.

Les résultats sont présentés en Figure 6.

Les cellules de levure traitées par le clofilium tosylate respirent mieux.

### Exemple 6 : Effet du clofilium tosylate sur la quantité de protéines Mip1 sauvages ou Mip1G651S mutées de levure

L'analyse de l'effet du clofilium tosylate sur la quantité de protéines Mip1 sauvages ou mutées est réalisée sur des extraits cellulaires enrichis en mitochondries. Afin de visualiser l'expression des protéines Mip1 et Mip1G651S, la séquence codant trois épitopes HA a été clonée en 3' et en phase avec la séquence codante de Mip1 et Mip1G651S.

Les cellules de levure sont mises en culture en milieu sélectif (SC-TRP, 2% Ethanol) pendant une nuit. Puis les cellules sont ensemencées à une densité optique (DO₆₀₀) initiale de 0,05 dans 100 mL de YPE (1% Yeast Extract, 0,5% Bacto Peptone, 2% Ethanol) supplémenté soit par du DMSO ou par du clofilium tosylate 32 µM à 28°C avec agitation jusqu'à une DO₆₀₀ comprise entre 3 et 4. Les cellules sont centrifugées à 3000 g pendant 5 min et lavées à l'eau. L'isolation de la fraction enrichie en mitochondries et l'extraction protéique de cette fraction sont réalisées selon le protocole décrit par Hoffman et al., 2009 (Hofmann, L., Saunier, R., Cossard, R., Esposito, M., Rinaldi, T. and Delahodde, A. (2009) A non-proteolytic proteasome activity controls organelle fission in yeast. J. Cell. Sci., 122, 3673 - 3682).

Les extraits protéiques des fractions enrichies en mitochondries sont analysés par électrophorèse sur gel SDS/PAGE 6% pour l'analyse des protéines Mip1-HA et Mip1G651S-HA et 10% pour l'analyse des protéines mitochondriales Cox2 (codée par l'ADNmt) et de la porine. Après séparation sur gel de polyacrylamide les protéines sont transférées sur une membrane de nitrocellulose. Après saturation des membranes par la solution de lait écrémé à 5 % (p/v), les membranes sont incubées pendant une heure sous agitation en présence des anticorps primaires correspondants (la protéine Mip1 : anti-HA dilués au 1/5000^{ème}, la protéine Cox2 : anti-Cox2 dilués au 1/500^{ème}, la porine : anti-porine dilués au 1/25000^{ème}). Après lavage en tampon TBS, les membranes sont incubées pendant une heure en présence des anticorps secondaires couplés à la *horseradish peroxydase* (HRP) dilués au 1/5000^{ème}. Puis les membranes sont relavées en tampon TBS. Les protéines sont ensuite révélées à l'aide du Kit de détection de l'activité peroxydase *ECL^{Plus}* de GE Healthcare.

Les résultats sont présentés en Figure 7.

La quantité de protéine Mip1 sauvage ou Mip1G651S mutée de levure est augmentée en présence de clofilium tosylate.

### Exemple 7 : Détermination de la concentration non-toxique de clofilium tosylate (MIC, Minimum Inhibitory Concentration) chez le nématode Caenorhabditis elegans

Une série de boîtes NGM contenant du clofilium tosylate aux concentrations de 1 µM, 5 µM, 10 µM, 50 µM, 100 µM ou 200 µM est préparée (trois boîtes pour chaque concentration). Entre 30 et 40 larves L1 synchronisées des lignées sauvage N2 BRISTOL ou mutée VC1224 (polg-1(ok1548Δ/+)/mT1 II; +/mT1[dpy-10(e128)] II), nématodes portant une délétion hétérozygote de 2149 bp dans la séquence codante du gène polg-1, sont déposées sur chacune de ces boîtes. Leur développement est observé pendant 4 jours.

Les résultats sont présentés en Figure 8.

Pour la lignée sauvage, un arrêt de développement au stade L3 ou un ralentissement de développement est observé à 100 µM de clofilium tosylate indiquant que la concentration maximale à utiliser est de 50 µM. Pour la souche polg-1(ok1548Δ/+), les larves se développent de manière similaire pour des concentrations allant de 1 µM à 50 µM de clofilium tosylate. La concentration en clofilium tosylate choisie pour les futures expériences chez le nématode est de 50 µM.

### Exemple 8: Effet du clofilium tosylate sur la résistance des nématodes polg-1 haploinsuffisant polg-1(ok1548Δ/+) au bromure d'éthidium

Le bromure d'éthidium est un agent intercalant de l'ADN, et plus spécialement de l'ADNmt, riche en adénine et thymidine. A forte concentration il induit un arrêt de développement au stade L3 des vers (Addo, M. G., Cossard, R., Pichard, D., Obiri-Danso, K., Rötig, A. and Delahodde, A. (2010) Caenorhabditis elegans, a pluricellular model organism to screen new genes involved in mitochondrial genome maintenance. BBA-Mol. Basis Dis., 1802, 765 - 773). Les nématodes TB2143 polg-1(ok1548Δ/+) haploinsuffisants sont fortement sensibles à de faibles doses de bromure d'éthidium (30 µg/mL) avec 90% des larves arrêtées au stade L3.

### 8.1. Test de résistance au bromure d'éthidium

Une trentaine de larves synchronisées au stade L1 sauvages ou mutées dans polg-1(lignée TB2143 polg-1(ok1548Δ/+)) est déposée sur des boîtes NGM contenant 30 µg/mL de bromure d'éthidium et soit du DMSO soit du clofilium tosylate 50 µM (expérience faite en triplicat). Le développement larvaire est examiné après 4 jours de traitement.

Les résultats sont présentés en Figure 9.

Le clofilium tosylate entraîne une résistance accrue des nématodes haploinsuffisants polg-1(ok1548Δ/+) au bromure d'éthidium.

### 8.2. Dose réponse du clofilium tosylate sur la résistance des nématodes haploinsuffisants polg-1(ok1548Δ/+) au bromure d'éthidium

Une trentaine de larves synchronisées au stade L1 mutées dans polg-1, lignée TB2143 polg-1(ok1548Δ/+), est déposée sur des boîtes NGM contenant 30 µg/mL de bromure d'éthidium et soit du DMSO soit différentes concentrations de clofilium tosylate : 1 µM, 5 µM, 10 µM et 50 µM (expérience faite en triplicat). Le développement larvaire est examiné après 4 jours de traitement.

La courbe dose-réponse est donnée en Figure 10.

La dose-réponse du clofilium tosylate est déterminée une nouvelle fois selon le même mode opératoire, avec des concentrations de clofilium tosylate de 0,1 µM, 0,5 µM, 1 µM, 5 µM, 10 µM et 50 µM.

Les résultats sont présentés en Figure 18.

### 8.3. Dose réponse de la quinidine (contrôle négatif), sur la résistance des nématodes haploinsuffisants polg-1(ok1548Δ/+) au bromure d'éthidium

Une trentaine de larves synchronisées au stade L1 mutées dans polg-1, lignée TB2143 polg-1(ok1548Δ/+), est déposée sur des boîtes NGM contenant 30 µg/mL de bromure d'éthidium et soit du DMSO soit différentes concentrations de quinidine, un antiarythmique de classe 1:10 µM, 30 µM, 100 µM et 300 µM (expérience faite en triplicat). Le développement larvaire est examiné après 4 jours de traitement.

La courbe dose-réponse est donnée en Figure 11.

### Exemple 9 : Effet du clofilium tosylate sur le nombre d'oeufs pondus et éclos des nématodes homozygotes TB2143 polg-1(Δ/Δ)

Quatre larves au stade L4 TB2143 polg-1(ok1548Δ/Δ) homozygotes sont déposées sur chaque boîte NGM contenant soit du DMSO soit du clofilium tosylate 50 µM (trois boîtes pour chaque groupe de traitement). Elles sont incubées à 20°C jusqu'au stade adulte. Toutes les 9 à 12 heures et ce pendant 6 à 7 jours, jusqu'à absence d'oeuf dans l'utérus, les adultes sont déplacés sur de nouvelles boîtes contenant du DMSO ou du clofilium tosylate 50 µM, et le nombre d'oeufs pondus est compté. Chaque ponte est suivie jusqu'au lendemain pour le dénombrement de l'éclosion.

Les résultats sont présentés en Figure 12.

Le clofilium tosylate augmente le nombre d'oeufs pondus et l'éclosion des embryons des nématodes homozygotes TB2143 polg-1(Δ/Δ).

### Exemple 10 : Effet du clofilium tosylate sur l'extrusion des gonades et/ou de l'intestin des nématodes homozygotes

Dix larves au stade L4 TB2143 polg-1(ok1548Δ/Δ) homozygotes sont déposées sur chaque boîte NGM contenant soit du DMSO soit du clofilium tosylate 50 µM (trois boîtes pour chaque groupe de traitement) et sont incubées à 20°C. Leur survie est suivie jusqu'au 7^{ème} jour de l'adulte. Le nombre d'animaux morts, ne répondant à aucune stimulation, et présentant une extrusion de l'intestin et/ou des gonades est compté. Les rares animaux morts de déshydratation sur le bord des boîtes de pétri sont exclus de l'analyse.

Les résultats sont présentés en Figure 13.

Le clofilium tosylate réduit la mort prématurée des nématodes homozygotes par extrusion des gonades et/ou de l'intestin.

### Exemple 11 : Effet du clofilium tosylate sur la quantité d'ADNmt des nématodes homozygotes TB2143 polg-1(ok1548Δ/Δ) déplétés en ADNmt

Dix larves au stade L4 TB2143 polg-1(ok1548Δ/Δ) homozygotes ou sauvages sont déposées sur chaque boîte NGM contenant soit du DMSO soit du clofilium tosylate 50 µM (trois boîtes pour chaque groupe de traitement). Elles sont incubées à 20°C jusqu'au 6^{ème} jour de l'adulte. L'extraction des ADN totaux des nématodes est réalisée avec le kit *Nucleospin Kit Tissue* (Macherey Nagel). Les quantifications de l'ADNmt et de l'ADN nucléaire sont réalisées comme décrit dans Addo et al., 2010 (Addo, M. G., Cossard, R., Pichard, D., Obiri-Danso, K., Rötig, A. and Delahodde, A. (2010) Caenorhabditis elegans, a pluricellular model organism to screen new genes involved in mitochondrial genome maintenance. BBA-Mol. Basis Dis., 1802, 765 - 773).

Les résultats sont présentés en Figure 14 et en Table 1.

**Table 1 : Rapport de la quantité d'ADNmt à la quantité d'ADN nucléaire par nématode sauvage N2 BRISTOL traité au DMSO et par nématode homozygote TB2143 polg-1(ok1548Δ/Δ) traité soit au DMSO soit avec du clofilium tosylate 50 µM (CLOF).**

| génotype | traitement | ADNmt/ADNnu |
|---|---|---|
| wild-type (N2) | DMSO | 488,9 |
| polg-1(ok1548Δ/Δ) | DMSO | 11,2 |
| polg-1(ok1548Δ/Δ) | CLOF | 69,2 |

Le clofilium tosylate augmente la quantité d'ADNmt des nématodes homozygotes TB2143 polg-1(ok1548Δ/Δ) déplétés en ADNmt.

### Exemple 12 : Détermination de la concentration maximale non toxique du clofilium tosylate sur des cultures de fibroblastes contrôles et de fibroblastes d'un patient avec une mutation sur chaque allèle du gène POLG

Les fibroblastes cutanés du contrôle et du patient sont cultivés en DMEM Galactose (DMEM dépourvu de glucose (Life Technologies) complémenté avec 10 mM de galactose, 10% de sérum de veau foetal, 200 U/mL de pénicilline, 200 U/mL de streptomycine). De l'uridine (50 µg/mL) et du pyruvate de sodium (2,5 mM) sont ajoutés au milieu de culture afin de maintenir les cellules exprimant un déficit de la chaîne respiratoire. Les cellules sont incubées en atmosphère contrôlée à 37°C et 5% de CO₂.

La prolifération cellulaire des fibroblastes du patient et du contrôle est mesurée en continu pendant 140 h par utilisation d'un automate xCELLigence (ACEA Biosciences) dans des plaques 96 puits. Le signal du bruit de fond est mesuré avec 50 µL de milieu de culture par puits. 50 µL de milieu contenant 1350 fibroblastes sont ensuite ajoutés dans chaque puits. La mesure d'impédance est réalisée toutes les minutes pendant 9 h puis toutes les 15 minutes jusqu'à la fin de l'analyse.

Le clofilium tosylate est dilué dans du DMSO 0,1% et utilisé à différentes concentrations (2,5 µM, 5 µM, 10 µM) dans un volume total de 50 µL. La détermination des concentrations toxiques du clofilium tosylate est réalisée en ajoutant la drogue dès l'ensemencement des cellules. Pour chaque concentration de drogue et chaque contrôle, l'analyse est réalisée en triplicat sur plaques de 96 puits. Un algorithme mathématique permet de convertir le signal d'impédance en index cellulaire (IC). Cet index est proportionnel au nombre de cellules adhérant au fond du puits ainsi qu'à leur forme.

Les résultats sont présentés en Figure 15.

La concentration maximale de clofilium tosylate autorisée sur les cultures de fibroblastes contrôles et de patient avec une mutation sur chaque allèle du gène POLG est de 2,5 µM.

### Exemple 13 : Effet du clofilium tosylate sur la quantité d'ADNmt chez les fibroblastes d'un patient avec une mutation sur chaque allèle du gène POLG cultivés en condition de prolifération cellulaire

Des fibroblastes cutanés du contrôle et du patient sont cultivés jusqu'à 100% de confluence dans un milieu *Dulbecco 's Modified Eagle Medium* (DMEM) haut glucose complet (4,5 g/L de D-glucose, 110 mg/L de sodium pyruvate, 10% de sérum de veau foetal, 1% de pénicilline/streptomycine à partir de la solution mère pénicilline 10000 IU et streptomycine 10000 µg/mL) à 37°C sous CO₂ 5%. A confluence, les cultures sont traitées à la trypsine et remises en culture à 30% de confluence dans un milieu DMEM haut glucose complet supplémenté soit avec du DMSO 0,1% soit avec 2,5 µM de clofilium tosylate. Elles sont incubées à 37°C sous CO₂ 5% et sont récoltées à 100% de confluence. Les ADN totaux des fibroblastes sont extraits par la méthode de phénol-chloroforme et la quantification des ADNmt et ADN nucléaire est réalisée par qPCR comme décrite dans Sarzi et al., 2007 (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

### Les résultats sont présentés en Figure 16.

En condition de prolifération cellulaire, le clofilium tosylate augmente légèrement la quantité d'ADNmt chez les fibroblastes du patient avec une mutation sur chaque allèle du gène POLG.

L'effet du clofilium tosylate sur la quantité d'ADNmt chez les fibroblastes d'un patient avec une mutation sur chaque allèle du gène POLG en condition de prolifération cellulaire cultivés dans un milieu DMEM glucose complet ou galactose complet est mesuré une seconde fois. Des fibroblastes cutanés du contrôle et du patient sont cultivés jusqu'à 100% de confluence dans un milieu DMEM glucose complet ou galactose complet (4,5 g/L de D-glucose ou 4,5 g/L de D-galactose, 110 mg/L de sodium pyruvate, 10% de sérum de veau foetal, 1% de pénicilline/streptomycine à partir de la solution mère pénicilline 10000 IU et streptomycine 10000 µg/mL) à 37°C sous CO₂ 5%. A confluence, les cultures sont traitées à la trypsine et remises en culture à 30% de confluence dans un milieu DMEM glucose complet ou galactose complet supplémenté soit avec du DMSO 0,1% soit avec du clofilium tosylate 2,5 µM. Elles sont incubées à 37°C sous CO₂ 5% et sont récoltées à 100% de confluence. Les ADN totaux des fibroblastes sont extraits par la méthode de phénol-chloroforme et la quantification des ADNmt et ADN nucléaire est réalisée par qPCR comme décrite dans Sarzi et al., 2007 (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

### Exemple 14 : Effet du clofilium tosylate sur la quantité d'ADNmt des fibroblastes d'un patient avec une mutation sur chaque allèle du gène POLG cultivés en condition de quiescence

Des fibroblastes cutanés du contrôle et du patient sont cultivés jusqu'à 100% de confluence dans du DMEM à 37°C sous CO₂ 5%. A 100% de confluence, les fibroblastes sont lavés au PBS et le milieu quiescent est ajouté (4,5 g/L de D-glucose, 110 mg/L de sodium pyruvate, 0,1% de sérum de veau foetal, 1% de pénicilline/streptomycine à partir de la solution mère pénicilline 10000 IU et streptomycine 10000 µg/mL). Après quatre jours en milieu quiescent, le clofilium tosylate 1 µM ou le DMSO (0,1%) sont ajoutés au milieu de culture. Le traitement est réalisé pendant 18 jours en changeant le milieu de culture avec ou sans clofilium tosylate tous les trois jours. Les fibroblastes sont récoltés tous les 6 jours et l'ADN total est extrait au phénol-chloroforme. L'ADNmt et l'ADN nucléaire sont quantifiés par qPCR (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

Les résultats sont présentés en Figure 17.

Le clofilium tosylate augmente la quantité d'ADNmt des fibroblastes du patient avec une mutation sur chaque allèle du gène POLG en condition de quiescence.

L'effet du clofilium tosylate sur la quantité d'ADNmt de fibroblastes d'un patient avec une mutation sur chaque allèle du gène POLG en condition de quiescence est mesuré une deuxième fois selon le même mode opératoire.

### Exemple 15 : Dose-réponse de l'Ibutilide sur la résistance des nématodes haploinsuffisants polg-1(ok1548Δ/+) au bromure d'éthidium

Des boîtes NGM supplémentées avec 30µg/mL de bromure d'éthidium et du DMSO ou 1 µM, 5 µM, 10 µM ou 50 µM d'Ibutilide Hemifumarate sont préparées (trois boîtes pour chaque groupe de traitement). Entre 30 et 40 larves de la souche TB2143 polg-1(ok1548Δ/+) synchronisées au stade L1 sont déposées sur chacune de ces boîtes. Leur développement est observé pendant 4 jours.

Les résultats sont présentés en Figure 19.

### Exemple 16 : Dose-réponse du Dofétilide sur la résistance des nématodes haploinsuffisants polg-1(ok1548Δ/+) au bromure d'éthidium

Des boîtes NGM supplémentées avec 30 µg/mL de bromure d'éthidium et du DMSO ou 1 µM, 5 µM, 10 µM, 50 µM, 100 µM ou 200 µM de Dofétilide ou 50 µM de clofilium tosylate sont préparées (trois boîtes pour chaque groupe de traitement). Entre 30 et 40 larves de la souche TB2143 polg-1(ok1548Δ/+) synchronisées au stade L1 sont déposées sur chacune de ces boîtes. Leur développement est observé pendant 4 jours.

Les résultats sont présentés en Figure 20.

### Exemple 17 : Effet du clofilium tosylate sur la production de colonies petite de la souche de levure mutante Δfis1

L'effet du clofilium est évalué selon le même mode opératoire que pour l'Exemple 2 avec des levures mutantes *Δfis1.*

Les résultats sont présentés en Figure 21.

Le produit du gène *FIS1,* impliqué dans le processus de fission des mitochondries, intervient dans le mécanisme d'action du clofilium tosylate.

### Exemple 18 : Elargissement de la dose-réponse du clofilium tosylate sur la quantité d'ADNmt de fibroblastes d'un patient avec une mutation sur chaque allèle du gène POLG cultivés en condition de quiescence

Des fibroblastes cutanés du contrôle et du patient sont cultivés jusqu'à 100% de confluence dans du DMEM à 37°C sous CO₂ 5%. A 100% de confluence, les fibroblastes sont lavés au PBS et le milieu quiescent est ajouté (4,5 g/L de D-glucose, 110 mg/L de sodium pyruvate, 0,1% de sérum de veau foetal, 1% de pénicilline/streptomycine à partir de la solution mère pénicilline 10000 IU et streptomycine 10000 µg/mL). Après quatre jours en milieu quiescent, le clofilium tosylate 30 nM, 100 nM, 300 nM ou 1 µM ou le DMSO (0,1%) sont ajoutés au milieu de culture. Le traitement est réalisé pendant 18 jours en changeant le milieu de culture avec ou sans clofilium tosylate tous les trois jours. Les fibroblastes sont récoltés tous les 6 jours et l'ADN total est extrait au phénol-chloroforme. L'ADNmt et l'ADN nucléaire sont quantifiés par qPCR (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

### Exemple 19 : Effet du clofilium tosylate sur la quantité d'ADNmt de fibroblastes d'un autre patient avec mutations différentes sur chaque allèle du gène POLG cultivés en condition de quiescence

Des fibroblastes cutanés du contrôle et du patient sont cultivés jusqu'à 100% de confluence dans du DMEM à 37°C sous CO₂ 5%. A 100% de confluence, les fibroblastes sont lavés au PBS et le milieu quiescent est ajouté (4,5 g/L de D-glucose, 110 mg/L de sodium pyruvate, 0,1% de sérum de veau foetal, 1% de pénicilline/streptomycine à partir de la solution mère pénicilline 10000 IU et streptomycine 10000 µg/mL). Après quatre jours en milieu quiescent, le clofilium tosylate 1 µM ou le DMSO (0,1%) sont ajoutés au milieu de culture. Le traitement est réalisé pendant 18 jours en changeant le milieu de culture avec ou sans clofilium tosylate tous les trois jours. Les fibroblastes sont récoltés tous les 6 jours et l'ADN total est extrait au phénol-chloroforme. L'ADNmt et l'ADN nucléaire sont quantifiés par qPCR (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

### Exemple 20 : Effet du clofilium tosylate sur un modèle de déplétion de l'ADNmt (non due à une mutation dans le gène POLG) avec mutation dans le gène orthologue d'OPA1 (eat-3) chez Caenorhabditis elegans.

### 20.1. Effet du clofilium tosylate sur les phénotypes du ver homozygote muté dans le gène eat-3 (eat-3(ad426) II ; him-8(e1489) IV) : Test de résistance au bromure d'ethidium

Une trentaine de larves synchronisées au stade L1 sauvages ou mutées dans eat-3 (lignée DA631 eat-3(ad426)) est déposée sur des boîtes NGM contenant différentes concentrations de bromure d'éthidium (10, 20, 30, 40, 50 µg/mL) et soit du DMSO soit du clofilium tosylate 50 µM (expérience en triplicat). Le développement larvaire est examiné après 4 jours de traitement.

### 20.2. Effet du clofilium tosylate sur le nombre d'oeufs pondus et éclos des nématodes DA631 et sur leur développement

Quatre larves au stade L4 DA631 (eat-3(ad426)) sont déposées sur chaque boîte NGM contenant soit du DMSO soit du clofilium tosylate 50 µM (trois boîtes pour chaque groupe de traitement). Elles sont incubées à 20°C jusqu'au stade adulte. Toutes les 9 à 12 heures et ce pendant 6 à 7 jours jusqu'à absence d'oeuf dans l'utérus, les adultes sont déplacés sur de nouvelles boîtes contenant du DMSO ou du clofilium tosylate 50 µM, et le nombre d'oeufs pondus est compté. Chaque ponte est suivie pour le dénombrement de l'éclosion et le développement larvaire.

### 20.3. Effet du clofilium tosylate sur la quantité d'ADNmt des nématodes DA631

Dix larves DA631 (eat-3(ad426)) au stade L4 ou sauvages sont déposées sur chaque boîte NGM contenant soit du DMSO soit du clofilium tosylate 50 µM (trois boîtes pour chaque groupe de traitement). Elles sont incubées à 20°C jusqu'au 6^{ème} jour de l'adulte. L'extraction des ADN totaux des nématodes est réalisée avec le kit *Nucleospin Kit Tissue* (Macherey Nagel). Les quantifications de l'ADNmt et de l'ADN nucléaire sont réalisées comme décrit dans Addo et al., 2010 (Addo, M. G., Cossard, R., Pichard, D., Obiri-Danso, K., Rötig, A. and Delahodde, A. (2010) Caenorhabditis elegans, a pluricellular model organism to screen new genes involved in mitochondrial genome maintenance. BBA-Mol. Basis Dis., 1802, 765 - 773).

### 20.4. Effet du clofilium tosylate sur la morphologie mitochondriale des nématodes DA631

Dix larves DA631 au stade L4 ou sauvages, exprimant la GFP adressée aux mitochondries dans les muscles locomoteurs, sont déposées sur boîte NGM contenant soit du DMSO soit du clofilium tosylate 50 µM. Elles sont incubées à 20°C jusqu'au 3^{ème} jour de l'adulte. Les adultes sont alors anesthésiés et mis entre lame et lamelle pour examen de la morphologie mitochondriale en microscopie à fluorescence.

### Exemple 21 : Effet de la combinaison d'administration de resveratrol (antioxydant) et de clofilium tosylate sur les fibroblastes d'un patient avec une mutation sur chaque allèle du gène POLG

### 21.1 : Effet sur la prolifération cellulaire

Les fibroblastes cutanés du contrôle et du patient sont cultivés en milieu DMEM Galactose (DMEM dépourvu de glucose (Life Technologies) complémenté avec 10 mM de galactose, 10% de sérum de veau foetal, 200 U/mL de pénicilline, 200 U/mL de streptomycine). De l'uridine (50 µg/mL) et du pyruvate de sodium (2,5 mM) sont ajoutés au milieu de culture afin de maintenir les cellules exprimant un déficit de la chaîne respiratoire. Les cellules sont incubées en atmosphère contrôlée à 37°C et 5% de CO₂. La prolifération cellulaire des fibroblastes du patient et du contrôle est mesurée en continu pendant 140 h par utilisation d'un automate xCELLigence (ACEA Biosciences) dans des plaques 96 puits. Le signal du bruit de fond est mesuré avec 50 µL de milieu de culture par puits. 50 µL de milieu contenant 1350 fibroblastes sont ensuite ajoutés dans chaque puits. La mesure d'impédance est réalisée toutes les minutes pendant 9 h puis toutes les 15 minutes jusqu'à la fin de l'analyse. Dès l'ensemencement des cellules, du clofilium tosylate (≤ 2 µM) ou du resveratrol (≤ 50 µM) ou une combinaison de clofilium tosylate (≤ 2 µM) et de resveratrol (≤ 50 µM) ou du DMSO sont ajoutés. Pour chaque drogue ou combinaison de drogues et chaque contrôle, l'analyse est réalisée en triplicat sur plaques de 96 puits. Un algorithme mathématique permet de convertir le signal d'impédance en index cellulaire (IC). Cet index est proportionnel au nombre de cellules adhérant au fond du puits ainsi qu'à leur forme.

### 21.2 : Effet sur la quantité d'ADNmt

Les fibroblastes cutanés du contrôle et du patient sont cultivés en milieu DMEM Galactose (DMEM dépourvu de glucose (Life Technologies) complémenté avec 10 mM de galactose, 10% de sérum de veau foetal, 200 U/mL de pénicilline, 200 U/mL de streptomycine). De l'uridine (50 µg/mL) et du pyruvate de sodium (2,5 mM) sont ajoutés au milieu de culture. Les cellules sont incubées en atmosphère contrôlée à 37°C et 5% de CO₂. A confluence, les cultures sont traitées à la trypsine et remises en culture à 30% de confluence dans un milieu DMEM galactose complet supplémenté soit avec du DMSO 0,1% soit avec du clofilium tosylate (≤ 2 µM), soit avec du resveratrol (≤ 50 µM), soit avec une combinaison de clofilium tosylate (≤ 2 µM) et de resveratrol (≤ 50 µM). Elles sont incubées à 37°C sous CO₂ 5% puis sont récoltées à 100% de confluence. Les ADN totaux des fibroblastes sont extraits par la méthode de phénol-chloroforme et la quantification des ADNmt et ADN nucléaire est réalisée par qPCR comme décrite dans Sarzi et al., 2007 (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

### Exemple 22 : Effet du clofilium tosylate sur le ratio des activités du complexe I / citrate synthase et la production de lactate de cybrides neuronaux dérivés d'un patient MELAS (m.3243A>G tRNA^{leu} mutation)

L'augmentation de la production de lactate est le reflet d'une toxicité de la drogue utilisée mais également d'un dysfonctionnement mitochondrial (déficit énergétique) présent dans le syndrome MELAS.

L'activité de la citrate synthase est un marqueur de masse mitochondriale et permet de normaliser l'activité du complexe I, qui est la première enzyme de la chaine respiratoire mitochondriale, qui est significativement réduite dans le syndrome MELAS.

La lignée transmitochondriale neuronale mutante MELAS, résultat de la fusion de la lignée parentale neuroblastome immortalisée SHSY-5Y avec des fibroblastes portant la mutation m.3243A>G a été créé dans le laboratoire du Pr Procaccio.

Les lignées neuronales cybrides porteuses de la mutation MELAS et la lignée parentale contrôle sont cultivées dans des flasques de culture stériles et mises à l'étuve à 37°C avec 5% de CO₂, dans un milieu composé de DMEM avec 1,5 g/L de glucose, 2 mM de L-glutamine et supplémenté de 10 % de sérum de veau foetal ainsi que d'uridine (5 µg/mL) et de pyruvate (10 µg/mL). Les cellules sont récoltées par trypsination en phase exponentielle et utilisées pour les analyses biochimiques ou moléculaires mitochondriales ultérieures.

La lignée neuronale cybride MELAS porteuse de la mutation en apparence à l'état homoplasmique (c'est-à-dire atteignant presque 100% de mutation MELAS) et la lignée parentale SHSY-5Y contrôle ont été utilisées pour les mesures d'activités enzymatiques. Les cybrides neuronaux en culture à confluence environ 50% ont été traités avec le véhicule (DMSO) ou à la concentration de 300 nM (CT 300nM) de clofilium tosylate. Les cellules sont ensuite incubées à 37°C sous CO₂ 5% et récoltées après 48 heures de traitement. Après traitement à la trypsine, les cellules cybrides sont centrifugées et congelées dans des tubes conservés à -80°C sous forme de culots cellulaires. Ces culots cellulaires sont ensuite analysés par spectrophotométrie avec la mesure des différentes activités biochimiques mitochondriales, en particulier l'activité de la citrate synthase et l'activité du complexe I de la chaîne respiratoire. La citrate synthase localisée dans la matrice mitochondriale catalyse la première réaction du cycle de Krebs. Le dosage de son activité est utilisé comme un témoin de la quantité de mitochondries.

Les activités enzymatiques du complexe I et de la citrate synthase ont été mesurées selon le protocole décrit dans Medjda *et al,* 2009 (Medja F, Allouche S, Frachon P, Jardel C, Malgat M, Mousson de Camaret B, Slama A, Lunardi J, Mazat JP, Lombès A. Development and implementation of standardized respiratory chain spectrophotometric assays for clinical diagnosis. Mitochondrion. 2009 Sep;9(5):331-9).

La concentration en lactate du milieu de culture des cellules neuronales cybrides et parentales a été déterminée par spectrophotométrie à l'aide d'un kit enzymatique (Bohringer) sur un appareil Hitachi-Roche (Roche Diagnostics GmbH). La production de lactate a été mesurée selon le protocole décrit dans Desquiret *et al,* 2012 (Desquiret-Dumas V, Gueguen N, Barth M, Chevrollier A, Hancock S, Wallace DC, Amati-Bonneau P, HenrionD, Bonneau D, Reynier P, Procaccio V. Metabolically induced heteroplasmy shifting and L-arginine treatment reduce the energetic defect in a neuronal-like model of MELAS (2012) Biochimica et Biophysica Acta Molecular Basis of Disease. 1822(6):1019-29).

Les résultats sont présentés en Figure 22.

Le clofilium tosylate augmente l'activité du complexe I sans augmenter la production de lactate pour une lignée cellulaire cybride porteuse de la mutation m.3243A>G pour le tRNA^{leu} responsable du syndrome MELAS.

### Exemple 23 : Effet dose de l'ibutilide sur le ratio des activités du complexe I / citrate synthase et la production de lactate de cybrides neuronaux dérivés d'un patient MELAS (m.3243A>G tRNA^{leu} mutation)

La lignée neuronale cybride MELAS porteuse de la mutation en apparence à l'état homoplasmique (c'est-à-dire atteignant presque 100% de mutation MELAS) et la lignée parentale SHSY-5Y contrôle ont été utilisées pour les mesures d'activités enzymatiques. Les cybrides neuronaux en culture à confluence environ 50% ont été traités avec le témoin (DMSO) ou aux concentrations de 1 µM (ibu 1 µM) ou 300 nM (ibu 300 nM) d'ibutilide. Les cellules sont ensuite incubées à 37°C sous CO₂ 5% et récoltées après 48 heures de traitement. Après traitement à la trypsine, les cellules cybrides sont centrifugées et congelées dans des tubes conservés à -80°C sous forme de culots cellulaires. Ces culots cellulaires sont ensuite analysés par spectrophotométrie avec la mesure des différentes activités biochimiques mitochondriales, en particulier l'activité de la citrate synthase et l'activité du complexe I de la chaîne respiratoire. La citrate synthase localisée dans la matrice mitochondriale catalyse la première réaction du cycle de Krebs. Le dosage de son activité est utilisé comme un témoin de la quantité de mitochondries.

Les activités enzymatiques ont été mesurées selon le protocole décrit dans Medjda *et al,* 2009 (Medja F, Allouche S, Frachon P, Jardel C, Malgat M, Mousson de Camaret B, Slama A, Lunardi J, Mazat JP, Lombès A. Development and implementation of standardized respiratory chain spectrophotometric assays for clinical diagnosis. Mitochondrion. 2009 Sep;9(5):331-9). La production de lactate a été mesurée selon le protocole décrit dans Desquiret *et al,* 2012 (Desquiret-Dumas V, Gueguen N, Barth M, Chevrollier A, Hancock S, Wallace DC, Amati-Bonneau P, Henrion D, Bonneau D, Reynier P, Procaccio V. Metabolically induced heteroplasmy shifting and L-arginine treatment reduce the energetic defect in a neuronal-like model of MELAS (2012) Biochimica et Biophysica Acta Molecular Basis of Disease. 1822(6):1019-29).

Les résultats sont présentés en Figure 23.

L'ibutilide augmente l'activité du complexe I et diminue la production de lactate pour une lignée cellulaire cybride porteuse de la mutation m.3243A>G pour le tRNA^{leu} responsable du syndrome MELAS.

### Exemple 24 : Effet du clofilium tosylate sur la quantité de protéine POLG dans des fibroblastes contrôles et des fibroblastes d'un patient avec une mutation sur chaque allèle du gène POLG en condition de prolifération cellulaire.

Des fractions protéiques d'extraits totaux de fibroblastes contrôles et de fibroblastes d'un patient avec une mutation sur chaque allèle du gène POLG cultivés en présence de DMSO 0,1% ou avec 0,5 µM ou 1,0 µM de clofilium tosylate sont analysées par électrophorèse.

Les fibroblastes cutanés du contrôle et du patient sont cultivés jusqu'à 100% de confluence dans un milieu *Dulbecco's Modified Eagle Medium* (DMEM) haut glucose complet (4,5 g/L de D-glucose, 110 mg/L de sodium pyruvate, 10% de sérum de veau foetal, 1% de pénicilline/streptomycine à partir de la solution mère pénicilline 10000 IU et streptomycine 10000 µg/mL) à 37°C sous CO₂ 5%. A confluence, les cultures sont traitées à la trypsine et remises en culture à 30% de confluence dans un milieu DMEM haut glucose complet supplémenté soit avec du DMSO 0,1% soit avec 0,5 µM ou 1,0 µM de clofilium tosylate. Les cellules sont ensuite incubées à 37°C sous CO₂ 5% et récoltées à 100% de confluence. Après traitement à la trypsine, les protéines totales des fibroblastes sont extraites et analysées par western blot. Les protéines étudiées sont la protéine POLG (analyse sur gel SDS/PAGE 6%), les protéines mitochondriales ATP5B et TFAM ainsi que la protéine cytosolique TUB3A comme contrôle de charge. 45 µg de protéines sont déposées et après séparation, les protéines sont transférées sur membrane PVDF puis révélées par détection de l'activité peroxydase ECL^{Plus} à l'aide d'anticorps primaires et d'anticorps secondaires couplés à la horseradish peroxydase (HRP).

Les résultats sont présentés en figure 24.

Le clofilium tosylate augmente la quantité des protéines mitochondriales POLG, TFAM et ATP5B.

### Exemple 25 : Dose réponse du clofilium tosylate sur la quantité d'ADNmt des fibroblastes d'un patient avec une mutation sur chaque allèle du gène POLG cultivés en condition de quiescence

Des fibroblastes cutanés du patient sont cultivés jusqu'à 100% de confluence dans du DMEM à 37°C sous CO₂ 5%. A 100% de confluence, les fibroblastes sont lavés au PBS et le milieu quiescent est ajouté (4,5 g/L de D-glucose, 110 mg/L de sodium pyruvate, 0,1% de sérum de veau foetal, 1% de pénicilline/streptomycine à partir de la solution mère pénicilline 10000 IU et streptomycine 10000 µg/mL). Après quatre jours en milieu quiescent, le clofilium tosylate à 0,5 µM, 1 µM, 2,5 µM ou le DMSO (0,1%) sont ajoutés au milieu de culture. Le traitement est réalisé pendant 18 jours en changeant le milieu de culture avec ou sans clofilium tosylate tous les trois jours. Les fibroblastes sont récoltés tous les 6 jours et l'ADN total est extrait au phénol-chloroforme. L'ADNmt et l'ADN nucléaire sont quantifiés par qPCR (Sarzi, E., Bourdon, A., Chrétien, D., Zarhrate, M., Corcos, J., Slama, A., Cormier-Daire, V., De Lonlay, P., Munnich, A. and Rötig, A. (2007) Mitochondrial DNA Depletion is a Prevalent Cause of Multiple Respiratory Chain Deficiency in Childhood. J Pediatr, 105, 531 - 534).

Les résultats sont présentés en Figure 25.

Le clofilium tosylate augmente la quantité d'ADNmt des fibroblastes du patient avec une mutation sur chaque allèle du gène POLG en condition de quiescence pour des concentrations de clofilium tosylate de 0,5 µM et 1 µM.

## Revendications

1. Composé de formule la : 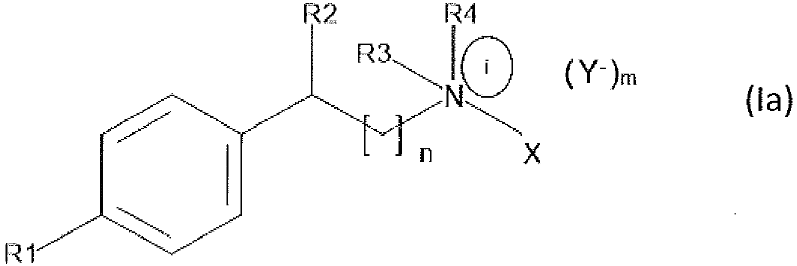 dans laquelle
R₁ représente un atome d'halogène, notamment Cl ou Br, un groupe alcoxy en C1-C4, un groupe alkyle en C1-C3 ou NHSO₂R₆;
R₂ représente OH ou H ;
R₃ et R₄ représentent indépendamment l'un de l'autre un groupe alkyle de 1 à 10 atomes de carbone ou 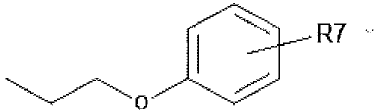 où R₇ représente un atome d'hydrogène, un atome d'halogène, un groupe alcoxy en C1-C4, un groupe alkyle en C1-C3 ou NHSO₂R₈ ;
R₆ et R₈ représentent indépendamment l'un de l'autre un groupe alkyle en C1-C4 ;
-X =-R₅ ou le doublet électronique de l'azote non engagé dans une liaison, sous réserve que i = + lorsque -X = - R₅ ;
R₅ représente un atome d'hydrogène ou un groupe alkyle en C1-C4;
m = 0 ou 1, sous réserve que m = 1 lorsque -X = -R₅ et m = 0 lorsque -X = le doublet électronique de l'azote non engagé dans une liaison ;
n = 0, 1, 2 ou 3 ;
Y⁻ est un anion acceptable sur le plan thérapeutique, en particulier Y" est choisi parmi l'ion tosylate, l'ion carbonate, l'ion phosphate et l'ion chlorure ;
pour son utilisation dans le traitement ou la prévention des maladies liées à l'instabilité de l'ADN mitochondrial.

2. Composé selon la revendication 1, de formule la : 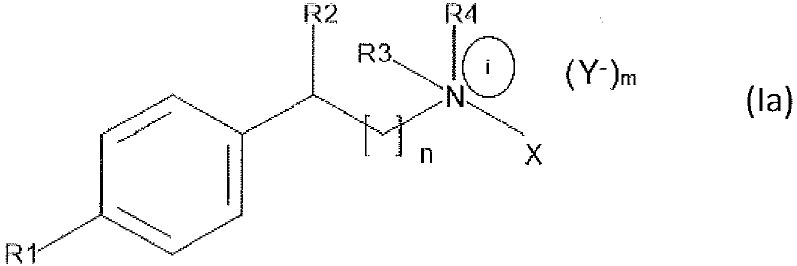 dans laquelle
R₁ représente Cl, Br, ou NHSO₂R₆; R₂ représente OH ou H ;
R₃ et R₄ représentent indépendamment l'un de l'autre un groupe alkyle de 1 à 10 atomes de carbone ou 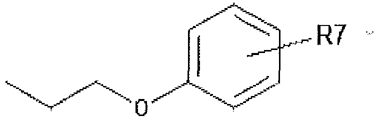 où R₇ représente un atome d'halogène ou NHSO₂R₈, sous réserve que R₃ et R₄ ne peuvent pas être simultanément 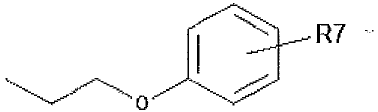
R₆ et R₈ représentent indépendamment l'un de l'autre un groupe alkyle en C1-C4 ;
-X =-R₅ ou le doublet électronique de l'azote non engagé dans une liaison, sous réserve que i = + lorsque -X = - R₅ ;
R₅ représente un groupe alkyle en C1-C4;
m = 0 ou 1, sous réserve que m = 1 lorsque -X = -R₅ et m = 0 lorsque -X = le doublet électronique de l'azote non engagé dans une liaison ;
n= 0, 1, 2 ou 3 ;
Y⁻ est choisi parmi l'ion tosylate, l'ion carbonate, l'ion phosphate et l'ion chlorure ;
pour son utilisation dans le traitement ou la prévention des maladies liées à l'instabilité de l'ADN mitochondrial.

3. Composé selon la revendication 1, de formule Iaa : 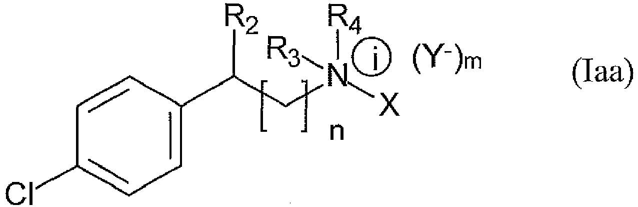 ou de formule Iab : 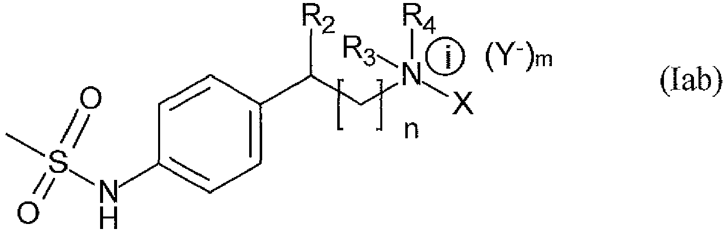
dans lesquelles R₂, R₃, R₄, X, R₅, 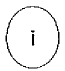 , Y⁻, m et n, ont les significations indiquées dans la revendication 1 ;
pour son utilisation dans le traitement ou la prévention des maladies liées à l'instabilité de l'ADN mitochondrial.

4. Composé selon la revendication 1, de formule (a) : 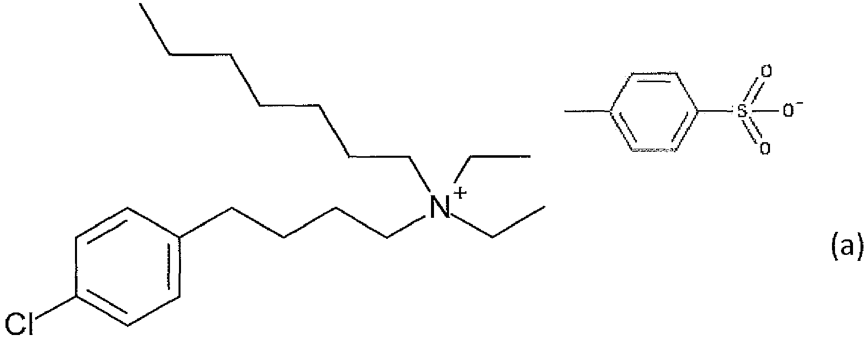 ou de formule (b) : 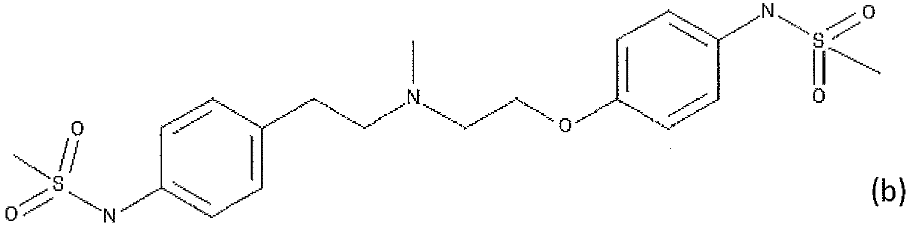 ou de formule (c) : 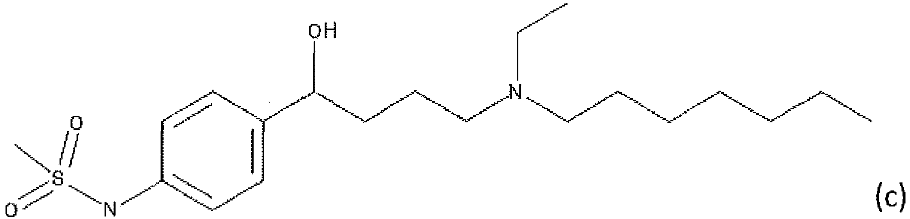 pour son utilisation dans le traitement ou la prévention des maladies liées à l'instabilité de l'ADN mitochondrial.

5. Composé selon la revendication 4, se présentant sous forme de dose unitaire de 30 µg à 6000 mg, pour son utilisation dans le traitement ou la prévention des maladies liées à l'instabilité de l'ADN mitochondrial.

6. Composé selon la revendication 4 ou 5, se présentant sous forme de dose unitaire de 30 µg à 6000 mg, pour une administration par voie orale,
ou se présentant sous forme de dose unitaire de 30 µg à 6000 mg, pour une administration par voie intraveineuse,
pour son utilisation dans le traitement ou la prévention des maladies liées à l'instabilité de l'ADN mitochondrial.

7. Composé selon la revendication 4, de formule (a): 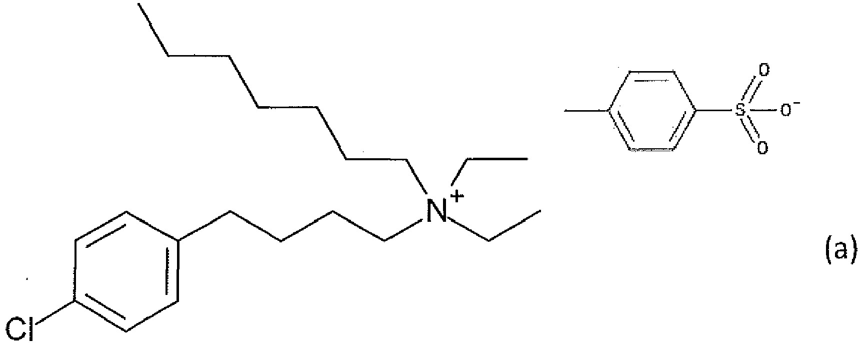
se présentant sous forme de dose unitaire de 1,5 mg à 60 mg pour une administration par voie orale,
ou se présentant sous forme de dose unitaire de 0,5 mg à 30 mg, pour une administration par voie intraveineuse,
pour son utilisation dans le traitement ou la prévention des maladies liées à l'instabilité de l'ADN mitochondrial.

8. Composé selon la revendication 4, de formule (b) : 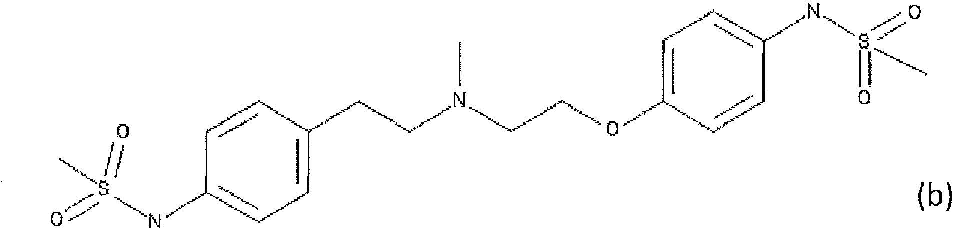 se présentant sous forme de dose unitaire de 125 à 500 µg, pour une administration par voie orale, pour son utilisation dans le traitement ou la prévention des maladies liées à l'instabilité de l'ADN mitochondrial.

9. Composé selon la revendication 4, de formule (c) : 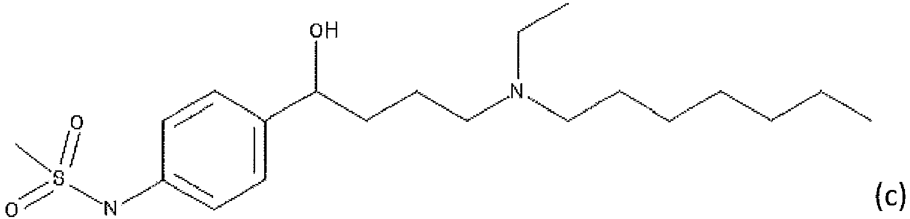 se présentant sous forme de dose unitaire de 150 µg à 2500 µg, pour une administration par voie intraveineuse, pour son utilisation dans le traitement ou la prévention des maladies liées à l'instabilité de l'ADN mitochondrial.

10. Composé selon l'une des revendications 1 à 9, pour son utilisation dans le traitement ou la prévention des maladies liées à au moins une mutation, ou au moins une délétion ou au moins une insertion, ou une combinaison de ces éléments, dans le gène POLG, ou des maladies liées à l'instabilité de l'ADN mitochondrial non liées aux mutations du gène POLG, ou des maladies liées à une déplétion ou une délétion de l'ADN mitochondrial, ou des maladies liées à une mutation ponctuelle de l'ADN mitochondrial.

11. Composé selon l'une des revendications 1 à 9, pour son utilisation dans le traitement ou la prévention
- de maladies associées à des anomalies quantitatives ou qualitatives de l'ADN mitochondrial telles que la maladie d'Alpers (AHS), le spectre des myo-cérébro-hépatopathies de l'enfant (MCHS), les épilepsies myocloniques avec myopathie et ataxie sensitive MEMSA (Myoclonic epilepsy myopathy sensory ataxia), l'ataxie spinocérébelleuse avec épilepsie (SCAE), les ataxies avec neuropathies incluant les syndromes MIRAS et SANDO, l'ophtalmoplégie externe progressive autosomique récessive (arPEO), l'ophtalmoplégie externe progressive autosomique dominante (adPEO), l'encéphalomyopathie mitochondriale neurogastrointestinale (MNGIE), le syndrome de Pearson, le syndrome de Kearns-Sayre, la myopathie infantile et l'amyotrophie spinale liées au mutations de *TK2,* les insuffisances hépatiques avec déplétion de l'ADNmt, les pathologies associées aux mutations des gènes *SUCLA2* et *SUCLAG1, RRM2B, AIF1, MPV17,* ou
- des maladies associées à des mutations ponctuelles de l'ADN mitochondrial telles que la neuropathie optique héréditaire de Leber, le syndrome MELAS, le syndrome MERRF, et certaines formes de syndrome de Leigh, d'ophtalmoplégie externe progressive chronique, de myopathie, de cardiomyopathie, de diabète-surdité, d'encéphalomyopathie et de surdité.

12. Composé selon l'une des revendications 1 à 11, pour son utilisation dans le traitement ou la prévention du syndrome MELAS.

## Patentansprüche

1. Verbindung der Formel Ia: 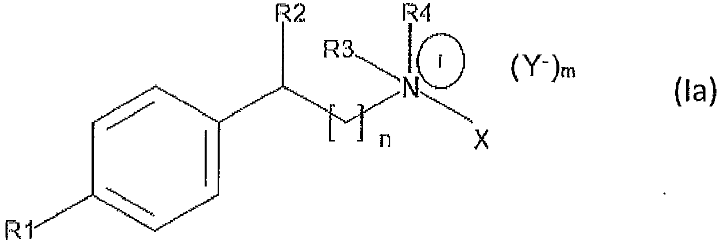 worin
R₁ für ein Halogenatom, insbesondere Cl oder Br, eine C1-C4-Alcoxygruppe, eine C1-C3-Alkylgruppe oder NHSO₂R₆ steht;
R₂ für OH oder H steht;
R₃ und R₄ unabhängig voneinander für eine Alkylgruppe mit 1 bis 10 Kohlenstoffatomen oder 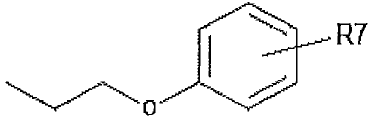 stehen, worin R₇ für ein Wasserstoffatom, ein Halogenatom, eine C1-C4-Alkoxygruppe, eine C1-C3-Alkylgruppe oder NHSO₂R₈ steht;
R₆ und R₈ unabhängig voneinander für eine C1-C4-Alkylgruppe stehen;
-X = -R₅ oder das Elektronenpaar von Stickstoff ist, das nicht an einer Bindung beteiligt ist, vorausgesetzt, dass i = + ist, wenn -X = -R₅ ist;
R₅ für ein Wasserstoffatom oder eine C1-C4-Alkylgruppe steht;
m = 0 oder 1 ist, vorausgesetzt, dass m = 1 ist, wenn -X = -R₅ ist und m = 0 ist, wenn -X = das Elektronenpaar von Stickstoff ist, das an keiner Bindung beteiligt ist;
n = 0, 1, 2 oder 3 ist;
Y⁻ ein therapeutisch verträgliches Anion ist, Y⁻ insbesondere ausgewählt ist aus dem Tosylation, dem Carbonation, dem Phosphation und dem Chloridion;
zur Verwendung bei der Behandlung oder der Vorbeugung von Krankheiten, die mit der Instabilität von Mitochondrien-DNA zusammenhängen.

2. Verbindung nach Anspruch 1 der Formel Ia: 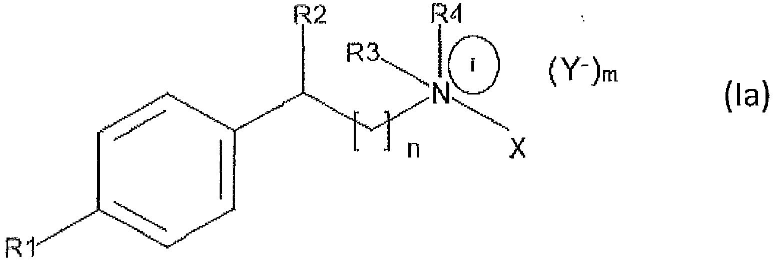 worin
R₁ für Cl, Br oder NHSO₂R₆ steht, R₂ für OH oder H steht;
R₃ und R₄ unabhängig voneinander für eine Alkylgruppe mit 1 bis 10 Kohlenstoffatomen oder 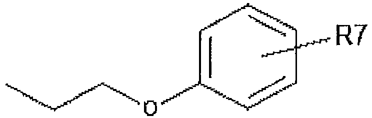 steht, worin R₇ für ein Halogenatom oder NHSO₂R₈ steht, vorausgesetzt, dass R₃ und R₄ nicht gleichzeitig 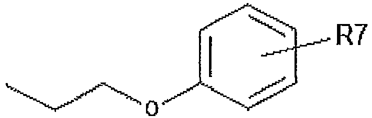 sein können,
R₆ und R₈ unabhängig voneinander für eine C1-C4-Alkylgruppe stehen;
-X = -R₅ oder das Elektronenpaar von Stickstoff ist, das nicht an einer Bindung beteiligt ist, vorausgesetzt, dass i = + ist, wenn -X = -R₅ ist;
R₅ für eine C1-C4-Alkylgruppe steht;
m = 0 oder 1 ist, vorausgesetzt, dass m = 1 ist, wenn -X = -R₅ ist und m = 0 ist, wenn -X = das Elektronenpaar von Stickstoff ist, das an keiner Bindung beteiligt ist;
n = 0, 1, 2 oder 3 ist;
Y⁻ ausgewählt ist aus dem Tosylation, dem Carbonation, dem Phosphation und dem Chloridion;
zur Verwendung bei der Behandlung oder der Vorbeugung von Krankheiten, die mit der Instabilität von Mitochondrien-DNA zusammenhängen.

3. Verbindung nach Anspruch 1 der Formel Iaa: 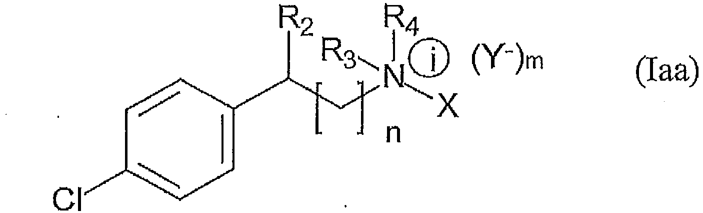 oder der Formel Iab: 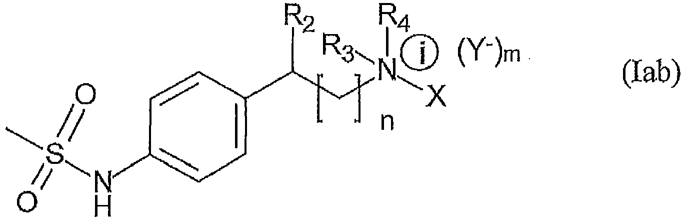
wobei R₂, R₃, R₄, -X, R₅ 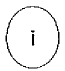 , Y⁻, m und n die gleichen Bedeutungen haben, wie in Anspruch 1 angegeben;
zur Verwendung bei der Behandlung oder der Vorbeugung von Krankheiten, die mit der Instabilität von Mitochondrien-DNA zusammenhängen.

4. Verbindung nach Anspruch 1 der Formel (a): 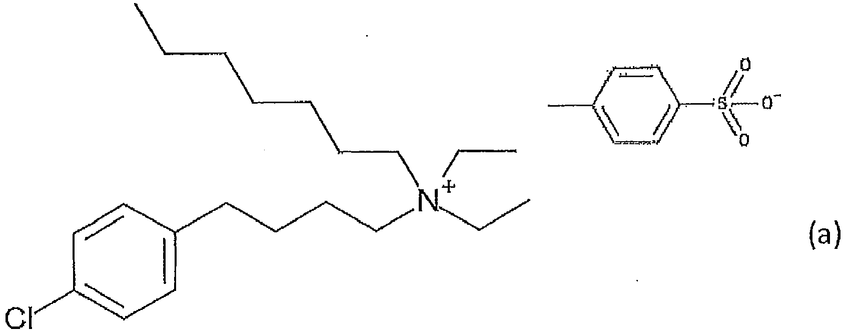 oder der Formel (b): 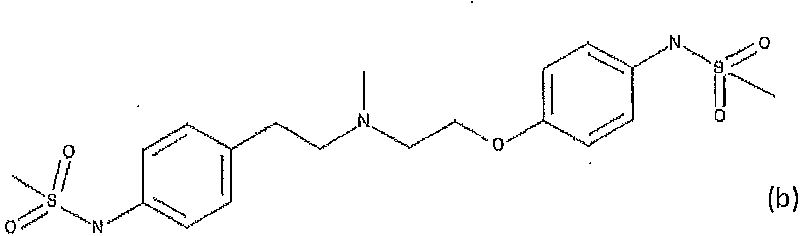 oder der Formel (c): 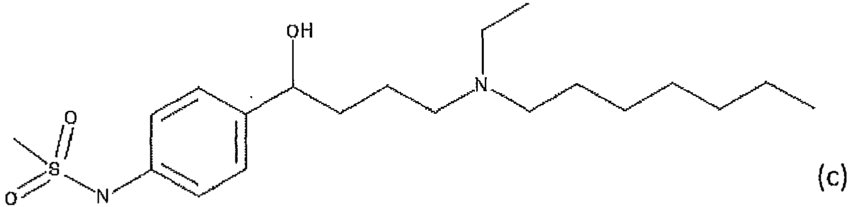 zur Verwendung bei der Behandlung oder der Vorbeugung von Krankheiten, die mit der Instabilität von Mitochondrien-DNA zusammenhängen.

5. Verbindung nach Anspruch 4, in Form einer Einheitsdosis von 30 µg bis 6.000 mg, zur Verwendung bei der Behandlung oder der Vorbeugung von Krankheiten, die mit der Instabilität von Mitochondrien-DNA zusammenhängen.

6. Verbindung nach Anspruch 4 oder 5 in Form einer Einheitsdosis von 30 µg bis 6.000 mg zur oralen Verabreichung
oder in Form einer Einheitsdosis von 30 µg bis 6.000 mg zur intravenösen Verabreichung zur Verwendung bei der Behandlung oder der Vorbeugung von Krankheiten, die mit der Instabilität von Mitochondrien-DNA zusammenhängen.

7. Verbindung nach Anspruch 4 der Formel (a): 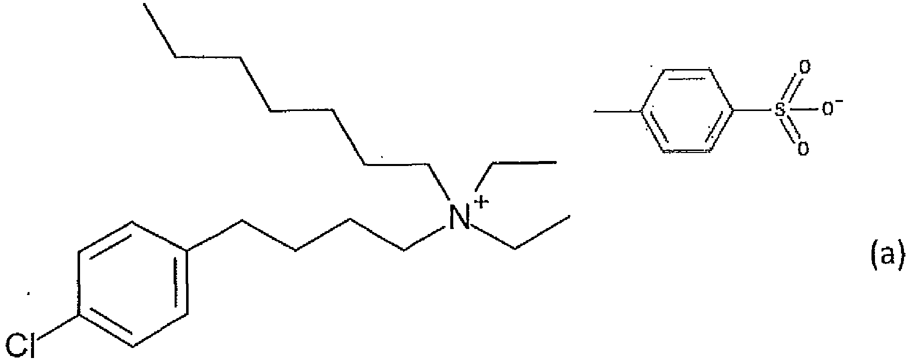
in Form einer Einheitsdosis von 1,5 mg bis 60 mg für eine orale Verabreichung,
oder in Form einer Einheitsdosis von 0,5 mg bis 30 mg für eine intravenöse Verabreichung,
zur Verwendung bei der Behandlung oder der Vorbeugung von Krankheiten, die mit der Instabilität von Mitochondrien-DNA zusammenhängen.

8. Verbindung nach Anspruch 4 der Formel (b): 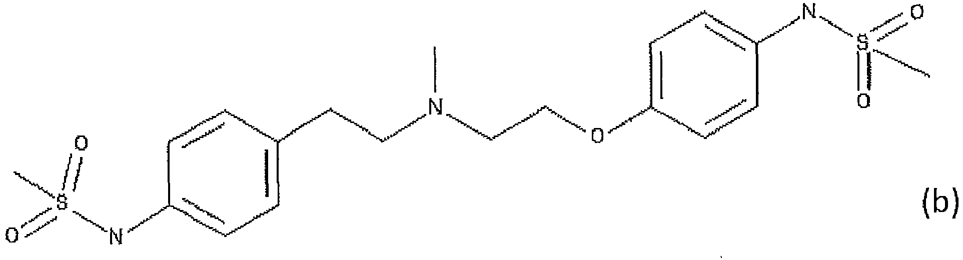 in Form einer Einheitsdosis von 125 bis 500 µg für eine orale Verabreichung, zur Verwendung bei der Behandlung oder der Vorbeugung von Krankheiten, die mit der Instabilität von Mitochondrien-DNA zusammenhängen.

9. Verbindung nach Anspruch 4 der Formel (c): 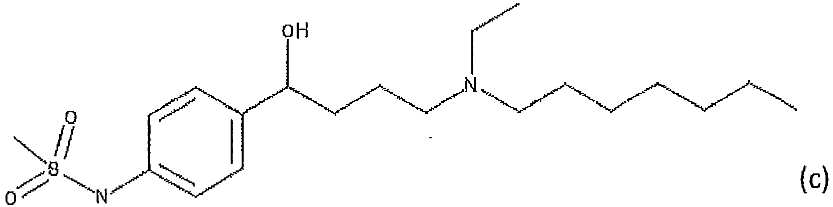 in Form einer Einheitsdosis von 150 µg bis 2.500 µg für eine intravenöse Verabreichung zur Verwendung bei der Behandlung oder der Vorbeugung von Krankheiten, die mit der Instabilität von Mitochondrien-DNA zusammenhängen.

10. Verbindung nach einem der Ansprüche 1 bis 9 zur Verwendung bei der Behandlung oder der Vorbeugung von Krankheiten, die mit mindestens einer Mutation oder mindestens einer Deletion oder mindestens einer Insertion oder einer Kombination dieser Elemente in dem Gen POLG zusammenhängen, oder Krankheiten, die mit der Instabilität der Mitochondrien-DNA zusammenhängen, die nicht mit den Mutationen des Gens POLG zusammenhängen, oder Krankheiten, die mit einer Depletion oder einer Deletion der Mitochondrien-DNA zusammenhängen, oder Krankheiten, die mit einer Punktmutation der Mitochondrien-DNA zusammenhängen.

11. Verbindung nach einem der Ansprüche 1 bis 9 zur Verwendung bei der Behandlung oder der Vorbeugung
- von Krankheiten, die mit quantitativen oder qualitativen Anomalien der Mitochondrien-DNA zusammenhängen, wie etwa dem Alpers-Syndrom (AHS), dem Myo-Zerebro-Hepatopathie-Spektrum im Kindesalter (MCHS), den Myoklonusepilepsien mit Myopathie und sensorischer Ataxie MEMSA (Myoclonic epilepsy myopathy sensory ataxia), der spinocerebellären Ataxie mit Epilepsie (SCAE), den Ataxien mit Neuropathien, einschließlich der Syndrome MIRAS und SANDO, der autosomalrezessiven progressiven Ophtalmoplegie (arPEO), der autosomal-dominanten progressiven Ophtalmoplegie (adPEO), der mitochondrialen neurogastrointestinalen Enzephalomyopathie (MNGIE), dem Pearson-Syndrom, dem Kearns-Sayre-Syndrom, der infantilen Myopathie und der mit Mutationen von TK2 assoziierten spinalen Muskelatrophie, den Leberfunktionsstörungen mit ADNmt-Depletion, den Pathologien, die mit Mutationen der Gene *SUCLA2* und *SUCLAG1, RRM2B, AIF1, MPV17* assoziiert sind, oder
- den Krankheiten, die mit Punktmutationen der Mitochondrien-DNA zusammenhängen, wie etwa der erblichen Optikus-Neuropathie Typ Leber, dem MELAS-Syndrom, dem MERRF-Syndrom und einigen Formen des Leigh-Syndroms mit externer chronischprogressiver Ophtalmoplegie, mit Myopathie, mit Kardiomyopathie, Diabetes-Taubheit, Enzephalomyopathie und Taubheit.

12. Verbindung nach einem der Ansprüche 1 bis 11 zur Verwendung bei der Behandlung oder der Vorbeugung des MELAS-Syndroms.

## Claims

1. Compound of formula la: 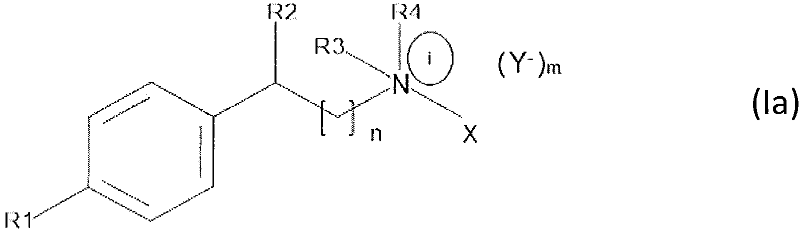 in which
R₁ represents a halogen atom, in particular Cl or Br, a C1-C4 alkoxy group, a C1-C3 alkyl group, or NHSO₂R₆;
R₂ represents OH or H;
R₃ and R₄ independently of one another represent an alkyl group having 1 to 10 carbon atoms or 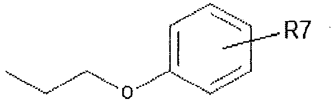 in which R₇ represents a hydrogen atom, a halogen atom, a C1-C4 alkoxy group, a C1-C3 alkyl group or NHSO₂R₈;
R₆ and R₈ independently of one another represent a C1-C4 alkyl group;
-X = -R₅ or the non-bonded electron pair of nitrogen, on the condition that i = + when -X = -R₅;
R₅ represents a hydrogen atom or a C1-C4 alkyl group;
m = 0 or 1, on the condition that m = 1 when -X = -R₅ and m = 0 when -X = the non-bonded electron pair of nitrogen;
n = 0, 1, 2 or 3;
Y⁻ is a therapeutically acceptable anion, in particular Y⁻ is selected from the tosylate ion, the carbonate ion, the phosphate ion, and the chloride ion;
for use in the treatment or prevention of diseases relating to the instability of mitochondrial DNA.

2. Compound according to claim 1, of formula la: 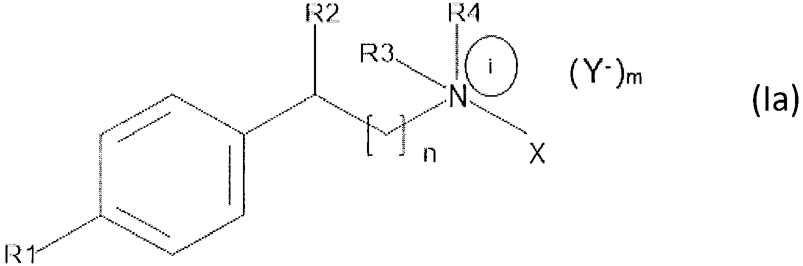 in which
R₁ represents Cl, Br, or NHSO₂R₆;
R₂ represents OH or H;
R₃ and R₄ independently of one another represent an alkyl group having 1 to 10 carbon atoms or 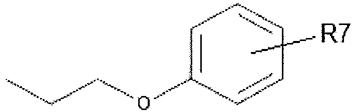
where R₇ represents a halogen atom or NHSO₂R₈, on the condition that R₃ and R₄ cannot simultaneously be 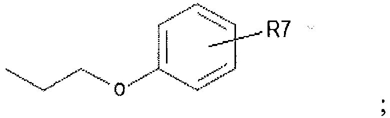 R₆ and R₈ independently of one another represent a C1-C4 alkyl group;
-X = -R₅ or the non-bonded electron pair of nitrogen, on the condition that i = + when -X =-R₅;
R₅ represents a C1-C4 alkyl group;
m = 0 or 1, on the condition that m = 1 when -X = -R₅ and m = 0 when -X = the non-bonded electron pair of nitrogen;
n = 0, 1,2 or 3;
Y⁻ is selected from the tosylate ion, the carbonate ion, the phosphate ion and the chloride ion; for use in the treatment or prevention of diseases relating to the instability of mitochondrial DNA.

3. Compound according to claim 1, of formula Iaa: 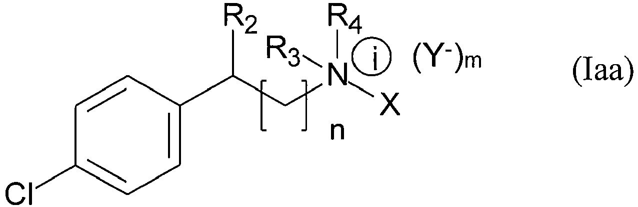 or of formula lab: 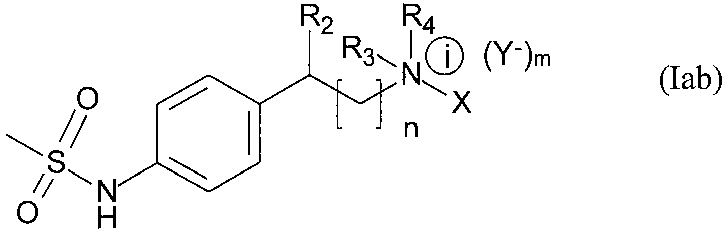 in which R₂, R₃, R₄, -X, R₅, 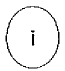 , Y⁻, m and n have the meanings indicated in claim 1;
for use in the treatment or prevention of diseases relating to the instability of mitochondrial DNA.

4. Compound according to claim 1, of formula (a): 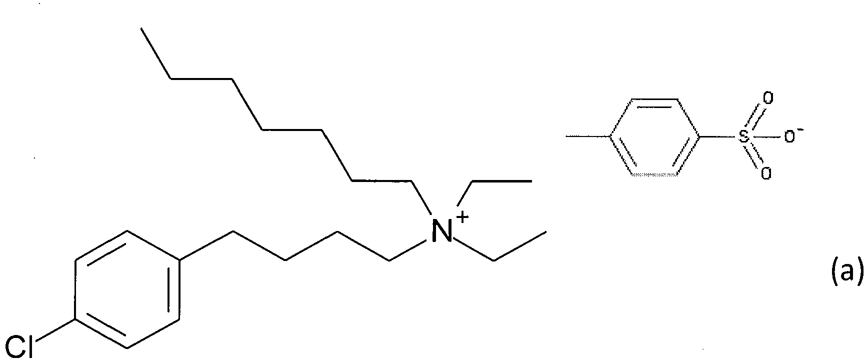 or of formula (b): 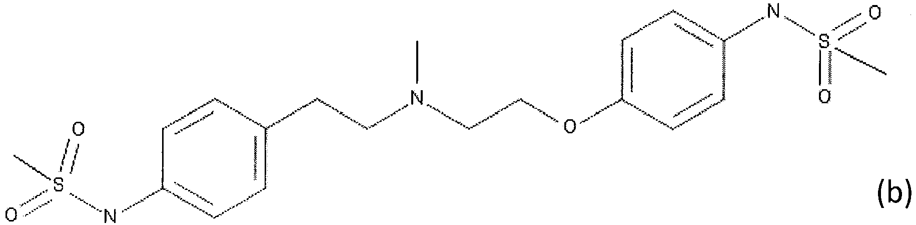 or of formula (c): 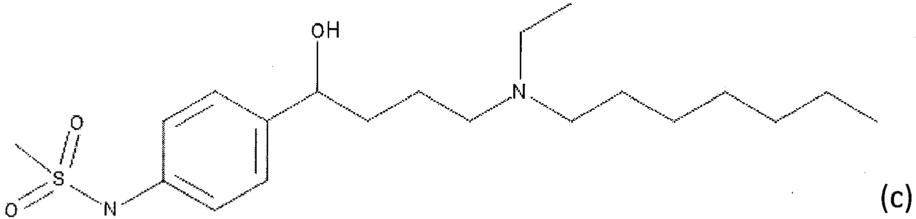 for use in the treatment or prevention of diseases relating to the instability of mitochondrial DNA.

5. Compound according to claim 4, present in the form of a unit dose of from 30 µg to 6000 mg, for use in the treatment or prevention of diseases relating to the instability of mitochondrial DNA.

6. Compound according to claim 4 or 5, under the form of a unit dose of from 30 µg to 6000 mg, for oral administration,
or under the form of a unit dose of from 30 µg to 6000 mg, for intravenous administration, for use in the treatment or prevention of diseases relating to the instability of mitochondrial DNA.

7. Compound according to claim 4, of formula (a): 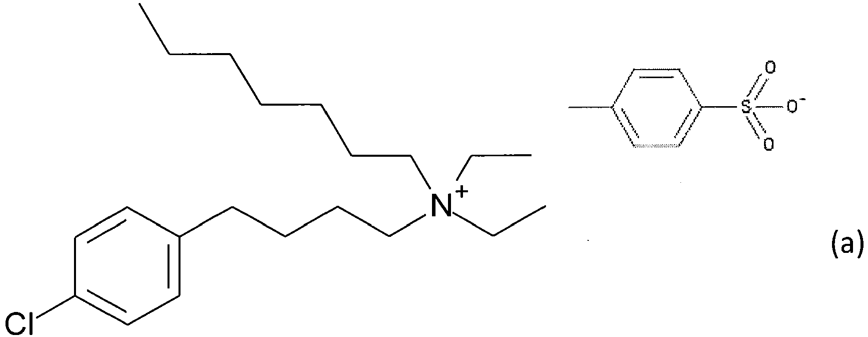
under the form of a unit dose of from 1.5 mg to 60 mg for oral administration,
or under the form of a unit dose of from 0.5 mg to 30 mg, for intravenous administration,
for use in the treatment or prevention of diseases relating to the instability of mitochondrial DNA.

8. Compound according to claim 4, of formula (b): 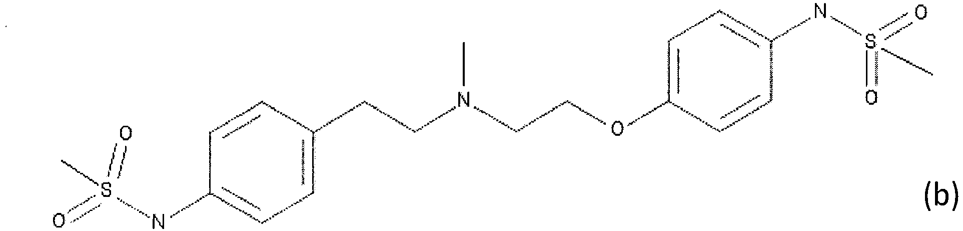 under the form of a unit dose of from 125 to 500 µg, for oral administration, for use in the treatment or prevention of diseases relating to the instability of mitochondrial DNA.

9. Compound according to claim 4, of formula (c): 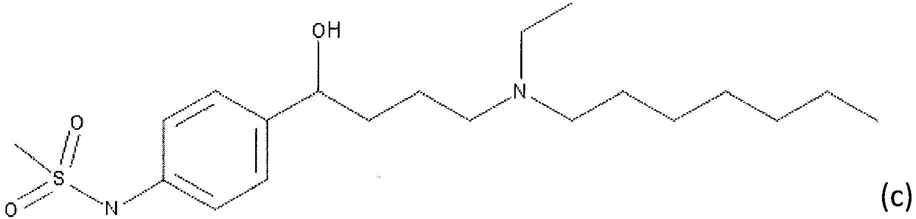 under in the form of a unit dose of from 150 to 2500 µg, for intravenous administration, for use in the treatment or prevention of diseases relating to the instability of mitochondrial DNA.

10. Compound according to any one of claims 1 to 9, for use in the treatment or prevention of diseases relating to at least one mutation, or at least one deletion or at least one insertion, or a combination thereof, in the POLG gene, or of diseases relating to the instability of mitochondrial DNA not associated with mutations of the POLG gene, or of diseases associated with a depletion or a deletion of mitochondrial DNA, or of diseases associated with a point mutation of mitochondrial DNA.

11. Compound according to any one of claims 1 to 9, for use in the treatment or prevention
- of diseases associated with quantitative or qualitative abnormalities of mitochondrial DNA, such as Alpers disease (AHS), childhood myocerebrohepatopathy spectrum (MCHS), myoclonic epilepsy myopathy sensory ataxia (MEMSA), spinocerebellar ataxia with epilepsy (SCAE), ataxia with neuropathy syndromes including MIRAS and SANDO, autosomal recessive progressive external ophthalmoplegia (arPEO), autosomal dominant progressive external ophthalmoplegia (adPEO), mitochondrial neurogastrointestinal encephalopathy (MNGIE), Pearson syndrome, Kearns-Sayre syndrome, infantile myopathy and spinal muscular atrophy relating to *TK2* mutations, liver failure with depletion of mtDNA, pathologies associated with mutations of the genes *SUCLA2* and *SUCLAG1, RRM2B, AIF1, MPV17,* or
- of diseases associated with point mutations of mitochondrial DNA, such as Leber hereditary optic neuropathy, MELAS syndrome, MERRF syndrome, and some forms of Leigh syndrome, chronic progressive external ophthalmoplegia, myopathy, cardiomyopathy, diabetes-deafness, encephalomyopathy, and deafness.

12. Compound according to any one of claims 1 to 11 for use in the treatment or prevention of MELAS syndrome.
